(19) Europäisches Patentamt
European Patent Office
Office européen des brevets

(11) EP 3 294 871 B1

(12) **EUROPEAN PATENT SPECIFICATION**

(45) Date of publication and mention of the grant of the patent:
**29.04.2026 Bulletin 2026/18**

(21) Application number: **16722215.7**

(22) Date of filing: **12.05.2016**

(51) International Patent Classification (IPC):
***C12N 5/078*** *(2010.01)* ***B01L 3/00*** *(2006.01)*

(52) Cooperative Patent Classification (CPC):
**B01L 3/502753; C12N 5/0644;** B01L 2200/0663; B01L 2300/0816; B01L 2300/0864; B01L 2300/0883; C12N 2500/36; C12N 2500/99; C12N 2501/125; C12N 2501/145; C12N 2501/727; C12N 2521/00; C12N 2533/50

(86) International application number:
**PCT/EP2016/060659**

(87) International publication number:
**WO 2016/180918 (17.11.2016 Gazette 2016/46)**

(54) **COMBINATION OF PHARMACOLOGICAL AND MICROFLUIDIC FEATURES FOR IMPROVED PLATELETS PRODUCTION**

KOMBINATION VON PHARMAKOLOGISCHEN UND MIKROFLUIDISCHEN FUNKTIONEN ZUR VERBESSERTEN HERSTELLUNG VON BLUTPLÄTTCHEN

COMBINAISON DE CARACTÉRISTIQUES MICROFLUIDIQUES ET PHARMACOLOGIQUES POUR AMÉLIORER LA PRODUCTION DE PLAQUETTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2015 EP 15167383**

(43) Date of publication of application:
**21.03.2018 Bulletin 2018/12**

(73) Proprietor: **HemostOD SA**
**1028 Préverenges (CH)**

(72) Inventors:
- **SICOT, Géraldine**
  **75006 Paris (FR)**
- **HAMDI, Feriel**
  **75006 Paris (FR)**
- **NIVAU, Audrey**
  **75006 Paris (FR)**

(74) Representative: **DTS Patent- und Rechtsanwälte PartmbB**
**Brienner Straße 1**
**80333 München (DE)**

(56) References cited:
**WO-A1-2010/063823 WO-A1-2015/075030**
**WO-A2-2012/129109**

- **ISABELLA PALLOTTA ET AL: "Three-Dimensional System for the In Vitro Study of Megakaryocytes and Functional Platelet Production Using Silk-Based Vascular Tubes", TISSUE ENGINEERING PART C: METHODS, vol. 17, no. 12, 1 December 2011 (2011-12-01), pages 1223 - 1232, XP055108904, ISSN: 1937-3384, DOI: 10.1089/ten.tec.2011.0134**
- **DUNOIS-LARDÉ C ET AL: "Exposure of human megakaryocytes to high shear rates accelerates platelet production", BLOOD, AMERICAN SOCIETY OF HEMATOLOGY, UNITED STATES, vol. 114, no. 9, 27 August 2009 (2009-08-27), pages 1875 - 1883, XP002563898, ISSN: 1528-0020, [retrieved on 20090612], DOI: 10.1182/BLOOD-2009-03-209205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- JINLIN JIANG ET AL: "Shear enhances thrombopoiesis and formation of microparticles that induce megakaryocytic differentiation of stem cells", 25 September 2014 (2014-09-25), pages 2094 - 2103, XP055227945, Retrieved from the Internet <URL:http://www.bloodjournal.org/content/bloodjournal/124/13/2094.full.pdf> [retrieved on 20151112], DOI: 10.1182/blood-2014-01-
- YOSUKE NAKAGAWA ET AL: "Two differential flows in a bioreactor promoted platelet generation from human pluripotent stem cell-derived megakaryocytes", EXPERIMENTAL HEMATOLOGY, vol. 41, no. 8, 1 August 2013 (2013-08-01), pages 742 - 748, XP055109130, ISSN: 0301-472X, DOI: 10.1016/j.exphem.2013.04.007
- SUZUKI AAE ET AL: "RhoA Is Essential for Maintaining Normal Megakaryocyte Ploidy Distribution and Platelet Generation", BLOOD, vol. 120, no. 21, November 2012 (2012-11-01), & 54TH ANNUAL MEETING AND EXPOSITION OF THE AMERICAN-SOCIETY-OF-HEMATOLOGY (ASH); ATLANTA, GA, USA; DECEMBER 08 -11, 2012, pages 385, XP009187166

**Description**

## FIELD OF THE INVENTION

The present invention relates to an ex vivo method for producing platelets including a combination of use of megakaryocyte modulator compounds and microfluidic device features suitable for high yield and high quality platelet production.

## BACKGROUND

Blood platelets are small anucleate cells that are crucial for the arrest of bleeding. There are many clinical diseases where platelets production or function is impaired. In addition, the number of patients who require platelet transfusion is increasing. Presently, the solution consists in conducting transfusions of platelets obtained from blood donors. Ex vivo platelet production for therapeutic applications is an appealing alternative, but remains a major technological challenge.

Platelets originate from megakaryocytes. Megakaryocyte differentiation is a continuous process characterized by sequential steps. While megakaryocyte differentiation takes place in the bone marrow, platelet production requires the passage of megakaryocyte fragments into the vessels of the bone marrow. Attempts of designing bioreactors specifically dedicated to platelet production have been made.

In order to produce platelets from megakaryocytes, different designs of the bioreactors were thought to reproduce the natural bone marrow environment.

Pallotta et al (Tissue Engineering: Part C, Vol. 17, n°12, 1223-1232, 2011) reported a 3D system that represents the first spatial reconstruction of the bone marrow environment. In this system, fragments of cells freely migrate from a scaffold in which they are embedded into a flowing channel through porosity or tubes. More recently, Di Buduo et al (Blood, Vol. 125, Issue 14, 2254-2264, 2015) performed a complete redesign of their previous model. They engineered a 3D bone marrow model made of porous silk to fully recreate the physiology of human bone marrow niche environment with improvement with respect to their previous model.

Nakagawa et al (Experimental Hematology, Vol. 41, n°8, 742-748, 2013) recently disclosed two bioreactors, which also mimic a capillary blood vessel for platelet production. In these systems, a flow in a specific direction applies a pressure and a shear stress and the megakaryocytes are trapped in a porous structure, where they release platelets. The platelets are collected at the outlet of the porous structure thanks to a flow in another direction.

Thon et al (Blood, Vol. 124, n°12, 1857-1867, 2014) described another bioreactor reproducing ex vivo bone marrow structure, including the presence of a wall pierced with slits. Megakaryocytes are pushed through the slits and brought into contact with the main flow and are exposed to a shear stress. In such systems, the number of slits available for megakaryocytes is limited, and many cells stay stuck in the reservoir while the first ones elongate and release platelets. This limits the speed of the platelet production process.

The designs of these bioreactors are not efficient for rapid large-scale production, in particular in view of the potential clogging of the megakaryocyte when passing through the porous walls. European patent application EP2014074905 describes another fluidic device for producing platelets from a suspension of megakaryocytes comprising a production chamber, composed of several channels in which a suspension of cells comprising megakaryocytes or their fragments can be introduced to flow from its inlet to its outlet. The channel of the fluidic device was advantageously featured with a tridimensional array of obstacles to increase megakaryocyte captures, without clogging. The same flow is applied to carry megakaryocytes through the device and to shear them leading to increased proplatelet formation and platelet production. Since megakaryocytes are able to continuously enter into the production chamber, there is no limitation to the production rate contrary to porous membrane systems of the prior art.

There is still a need to improve the yield of such in vitro production system.

The starting material for producing platelets is a suspension of cells comprising mature megakaryocytes. Prior art has described the effect of certain pharmacological substances in increasing polyploidization and proplatelet formation, when such substances are added in the culture medium during the expansion and the differentiation of megakaryocytes. For example, Chang et al (Blood, Vol. 109, n°10, 4229-4236, 2007) suggested that myosin light chain 2 (MLC2) phosphorylation is regulated by both ROCK and MLC kinase and plays an important role in platelet biogenesis by controlling proplatelet formation and fragmentation. Avanzi et al (British Journal of Haematology, Vol. 164, 867-876, 2014) confirmed that ROCK inhibition promotes demarcation membrane system (DMS) formation, and increases proplatelet formation and platelet release late in static megakaryocyte maturation.

However, contrary to the present invention, an effect of such pharmacological substances has never been observed during in vitro perfusion of mature megakaryocytes in a fluidic device under shear stress conditions.

The inventors have surprisingly found that compounds previously known to act in the polyploidization of megakaryocyte precursors, had also an effect on the quantity and quality of the produced platelets, when added only

at the perfusion stage in an ex vivo method for producing platelet, said method comprising perfusing the suspension of megakaryocytes in a fluidic device under shear stress conditions. The yield of produced platelets is significantly improved. Additionally, the platelets obtained by the ex vivo method using such megakaryocyte modulator compounds resemble more closely to human platelets.

These effects of such compounds at the perfusion stage are distinct from the polyploidization effect observed at the culture stage during expansion and differentiation of the megakaryocyte precursors.

## SUMMARY

The present invention provides an ex vivo method for producing platelets from megakaryocytes, as defined in claim 1.

Further advantageous implementations of the method are defined in the dependent claims.

The invention first relates to an ex vivo method for producing platelets from megakaryocytes, said method comprising:

a) providing a fluidic device comprising a production chamber including at least one channel delimited by (i) non-porous walls, (ii) one inlet opening at one end and (iii) one outlet opening at the other hand,
b) introducing a suspension of cells comprising megakaryocytes or their fragments into the inlet opening of the channel,
c) subjecting said suspension of cells to a flow from the inlet to the outlet of said channel, under a shear rate suitable for elongation and fragmentation of the megakaryocyte and platelet release in said channel,
d) collecting platelets at the outlet of the channel,

wherein at least one megakaryocyte modulator compound is added to said suspension of cells, said at least one megakaryocyte modulator compound being
selected from:

(i) an inhibitor of the Rho/ROCK pathway capable of increasing megakaryocytes ploidy,
(ii) nicotinamide,
(iii) blebbistatin,
(iv) latrunculin, and
(v) GM6001.

In another specific embodiment, said channel of the fluidic device is coated with a ligand with binding affinity for megakaryocytes, e.g., (i) von Willebrand factor (VWF) or its functional variants, (ii) polypeptides comprising fragments of von Willebrand factor, (iii) fibrinogen or (iv) fibronectin.

Preferably, said suspension of cells of megakaryocytes or their fragments for use in the above method of the invention is obtained by the following steps:

a) providing megakaryocyte progenitor and/or stem cells, for example $CD34^+$ cells from human umbilical cord blood,
b) culturing said megakaryocyte progenitor and/or stem cells for expanding the cells and differentiating the expanded cells into megakaryocytes,
c) washing the cells to remove the culture medium,
d) re- suspending the cells in a perfusion medium,

and said megakaryocyte modulator compound is added after the washing step c).

In one specific embodiment that may be combined with any of the preceding embodiments, the cells are collected at the outlet of the channel and reintroduced into the inlet of the channel for re-perfusion in the channel and platelet production. In one specific embodiment combined with the preceding embodiment, said collected cells are cultured for further maturation into megakaryocyte prior to reintroduction into the inlet of the channel

In another specific embodiment that may be combined with any of the preceding embodiments, said channel of the fluidic device has a substantially square or rectangular section. In a more specific embodiment of the previous embodiment, said channel has a height H that is comprised between 5 μm and 1 mm, preferably comprised between 25 μm and 100 μm and a width comprised between 100 μm and 5 mm, preferably comprised between 300 μm and 800 μm.

In another specific embodiment, said production chamber comprises a plurality of parallel channels, for example between 2 channels and $10^6$ channels. In another specific embodiment that may be combined with any of the preceding embodiments, said suspension of cells is subjected to a flow in the channel under a shear rate of at least 300 $s^{-1}$, preferably at least 600 $s^{-1}$, and more preferably at least 1000 $s^{-1}$, for example between 300 $s^{-1}$and 5000 $s^{-1}$ or between 600 $s^{-1}$ and

2400 $s^{-1}$.

In one example, a method may comprise:

a) providing a fluidic device comprising a production chamber including at least one channel delimited by (i) non-porous walls, (ii) at least one inlet opening at one end and (iii) at least one outlet opening at the other end; wherein at least one portion of the inner surface of the walls of said channel is textured with a plurality of obstacles,

b) introducing a suspension of cells comprising megakaryocytes or their fragments into the inlet opening of the channel,

c) subjecting said suspension to a flow from the inlet to the outlet of said channel, whereby the obstacles of said channel generate a shear rate suitable for elongation and fragmentation of the megakaryocyte and platelet release in said channel,

d) collecting platelets at the outlet of the channel,

wherein said megakaryocyte modulator compound is added to said suspension of cells.

The density, size and shape of the obstacles may be determined so as to enable the capture of megakaryocytes on said textured portion of the inner surface of the channel for platelet shedding.

For example, said obstacles may be posts or beams. For example, the obstacles may be posts with a substantially circular section of a radius r, and said posts may be arranged on the inner surface of at least one portion of said channel to form a regular pattern with a hexagonal periodic structure such as:

(i) the radius of a post r is preferably between 50 nm and 15 mm, more preferably between 500 nm and 1.5 mm,
(ii) the closest distance p between two post centers is at least equal to 100 nm, preferably between 100 nm and 50 mm, preferably between 500 nm and 10 mm, even better between 5 μιη and 1 mm,
(iii) the angle a, which is the smallest angle defined by the longitudinal direction of the channel and one of the lattice vectors of the hexagonal Bravais lattice is such as a is comprised between 0 and 90° and preferably between 0 and 30°
(iv) optionally said posts have a height h such as $0 < h < H$, wherein H refers to the smallest distance measured between two opposite walls in a section of the channel.

In a further example, said channel may be textured with a plurality of obstacles wherein the longitudinal and/or transversal distances between two obstacles decrease with respect to the flow direction to result in an increased density of the obstacles within the channel.

In another example, an ex vivo method for producing platelets from megakaryocytes, may comprise:

a) providing a suspension of cells comprising megakaryocytes or their fragments, b) introducing said suspension of cells in a channel of a fluidic device, c) subjecting said suspension of cells in the channel to a flow having a shear rate suitable for elongation and fragmentation of the megakaryocyte and platelet release, wherein the cells are collected at the outlet of the channel and reintroduced into the inlet of the channel for platelet production.

The method may further involve a recirculating step of the cells, said collected cells being cultured for further maturation into megakaryocyte prior to reintroduction into the inlet of the channel.

A fluidic device for producing platelets from a suspension of cells comprising megakaryocytes or their fragments, may comprise a production chamber including at least one channel delimited by (i) non-porous walls, (ii) at least one inlet opening at one end and (iii) at least one outlet opening at the other end; wherein at least one portion of the inner surface of the walls of said channel is textured with a plurality of obstacles, the density, size and shape of said obstacles are determined so as to enable the capture of megakaryocyte on said textured portion of the inner surface of the channel for platelet shedding, and wherein the longitudinal and/or transversal distances between two obstacle decreases with respect to the flow direction flow from the inlet to the outlet of the channel, to result in an increased density of the obstacles.

## DETAILED DESCRIPTION

The invention relates to an ex vivo method for producing platelets from megakaryocytes or their fragments, said method comprising:

(a) providing a fluidic device comprising a production chamber including at least one channel delimited by (i) non-porous walls, (ii) one inlet opening at one end and (iii) one outlet opening at the other end,

- introducing said suspension of cells comprising megakaryocytes or their fragments into the inlet opening of the channel,

subjecting said suspension to a flow from the inlet to the outlet of said channel, under a shear rate suitable for elongation and fragmentation of the megakaryocyte and platelet release in said channel, wherein at least one megakaryocyte modulator compound is added to said suspension of cells, said megakaryocyte modulator compound being a compound which is involved directly or indirectly in increasing megakaryocytes ploidy and/or involved in cytoskeleton organization.

As used herein, the term "platelets" denotes the anucleate cells that are involved in the cellular mechanisms of primary hemostasis leading to the formation of blood clots.

As used herein, the term "proplatelets" denotes any structural form of a megakaryocyte or its fragments, such as cytoplasmically-linked platelet-like particles, that could result in platelet formation. The structural forms include, but are not limited to, cells with long cytoplasmic extensions, projections or pseudopodia that contain swellings encompassing platelet bodies in various stages of formation, such as, nodules, beads, and the like. As used herein, the term "megakaryocyte" denotes a bone marrow cell responsible for the production of blood platelets necessary for normal hemostasis. Megakaryocytes are derived from hematopoietic progenitors restricted to the megakaryocytic lineage. The primary signal for megakaryocyte production is thrombopoietin or TPO. TPO is necessary for inducing differentiation of progenitor cells in the bone marrow towards a final megakaryocyte phenotype. Other molecular signals for megakaryocyte differentiation include for example GM-CSF, IL-3, IL-6, IL-11, Flt-3 ligand and SCF. Megakaryocyte progenitor cells can be obtained by in vitro culture.

The ex vivo method for producing platelets may be characterized by two main stages:
A first stage, which is called hereafter "the culture stage", consisting of culturing megakaryocyte progenitor and/or stem cells for expansion, differentiation and maturation into megakaryocytes.

A second stage, which is called hereafter "the perfusion stage", consisting of perfusing the suspension of cells comprising megakaryocytes or their fragments in the fluidic device for platelet release.

Typically, after the culture stage, the cells are washed to remove the culture medium and resuspended in the perfusion medium.

The perfusion stage aims at elongating and fragmenting the megakaryocytes and releasing platelets from them.

**Preparing the suspension of cells comprising megakaryocytes for use in the method for producing platelets**

A suspension of cells comprising megakaryocytes for use in the method for producing platelets is preferably obtained by the following steps:

(i) providing megakaryocyte progenitor and/or stem cells, for example $CD34^+$ cells from umbilical cord blood,
(ii) culturing said megakaryocyte progenitor and/or stem cells for expanding the cells and differentiating the expanded cells into megakaryocytes,
(iii) optionally, washing the cells to remove the culture medium, and resuspending the cells in a perfusion medium.

Regarding the above step (i), the megakaryocyte progenitor cells may typically be selected from hematopoietic stem cells (for example, from umbilical cord, peripheral blood or bone marrow), or from stem cells selected from the group consisting of embryonic stem cells and induced pluripotent stem (iPS) cells.

The above step (ii) aims at obtaining a sufficient number of mature megakaryocytes or their fragments for use in the perfusion stage and constitutes the essential step of the culture stage.

Details for the production of megakaryocytes from progenitors or stem cells may be found for example in Balduini et al (J Thromb Haemost, Vol. 6, 1900-1907, 2008) or in Takayama et al (The Journal of Experimental Medicine, Vol. 207, 2817-2830, 2010). More recently, Nakamura et al (Cell Stem Cell, Vol. 14, 1-14, 2014) established a new culture improvement from iPS cells with a stable immortalized megakaryocyte progenitor cell lines able to expand in culture over extended periods even after cryopreservation. Feng et al (Stem Cell Reports, Vol. 3, 1-15, 2014) developed a renewable cell source consisting on producing megakaryocytes from iPS cells in a scalable manner. Their method also permits the cryopreservation of megakaryocytes progenitors.

The culture stage may last for more than 24 hours, for example between 2 days and 8 weeks.

In specific embodiment, one or more megakaryocyte modulator compound as described in the next section is added in the culture medium at the culture stage for improving megakaryocyte ploidy and/or static proplatelets production as shown for example in Avanzi et al (British Journal of Haematology, Vol. 164, 867-876, 2014). In other embodiments, no megakaryocyte modulator compound is added at the culture stage.

Typically, at step (iii) of the above method, the cell population comprising megakaryocytes to be perfused in the fluidic device may be washed (to remove most of the constituents of the culture medium, for example by centrifugation and discarding of the culture medium), and resuspended in an appropriate perfusion medium, optionally comprising at least

one megakaryocyte modulator compound as described in the next section. In one embodiment, said perfusion medium is Iscove's Modified Dulbecco's Medium (IMDM) filtered, optionally supplemented with α-monothioglycerol and liposomes, and optionally supplemented with at least one megakaryocyte modulator compound as described in the next section.

The suspension for use in the perfusion stage preferably comprises a majority of megakaryocytes but may also comprise fragments of megakaryocytes or proplatelets, further to megakaryocytes.

In some embodiments, though a majority of the cells of the suspension may be megakaryocytes, other cells or cell residues may be found in minor amounts in such suspension, including without limitation, megakaryocyte progenitor cells, cytoplasmic fragments and proplatelets. In one embodiment, the suspension of cells comprising megakaryocytes for use in the devices or methods according to the invention thus further comprise fragments of megakaryocytes, and in particular proplatelets or platelets. For example, a majority of cells comprising megakaryocytes may express CD41 and CD42b antigens on their membrane.

In one embodiment, the suspension of cells comprising megakaryocytes which is introduced in the device presents a cell concentration comprised between $10^3$ and $10^8$ per mL, preferably between $10^5$ and $50.10^6$ per mL, within the perfusion medium.

**Supplementing the suspension of cells with at least one megakaryocyte modulator compound**

The inventors have found that compounds known to be involved directly or indirectly in megakaryocyte ploidy and/or involved in cytoskeleton organization under culture conditions have also the capacity to increase platelet production when supplemented at the perfusion stage.

Accordingly, the ex vivo method for producing platelet includes a step of adding at least one megakaryocyte modulator compound to said suspension of cells to be perfused in a fluidic device under shear stress conditions.

One or more distinct megakaryocyte modulator compounds may be added simultaneously or sequentially to the suspension of cells.

An efficient concentration of said one or more megakaryocyte modulator compounds is added into the suspension of cells to enable significant increase in the number of platelets released per megakaryocyte circulating in the channel of the fluidic device. The skilled person will therefore adapt the concentration of each megakaryocyte modulator compound depending of a number of factors including, without limitation, the concentration of megakaryocytes in the perfusion medium, the geometry of the fluidic device, the flow rate etc...

For example, an efficient concentration between 0.05 μM and 1 mM of a Rho/ROCK inhibitor (e.g Y27632 compound), more preferably between 2 μM and 50 μM, may be added to the suspension of cells. In another embodiment, an efficient concentration between 100 μM and 100 mM of a sirtuin inhibitor (e.g nicotinamide), more preferably between 2 mM and 50 mM, may be added to the suspension of cells. In another embodiment, an efficient concentration between 0.05 μM and 2 mM of a NMM II ATPase inhibitor (e.g blebbistatin), more preferably between 0.5 μM and 150 μM, may be added to the suspension of cells. In another embodiment, an efficient concentration between 1 nM and 100 mM of microtubule organizing regulators, more preferably between 1 μM and 2 mM, may be added to the suspension of cells. In another embodiment, an efficient concentration between 0.05 μM and 1 mM of cytoskeletal signaling inhibitors (e.g. Latrunculin), more preferably between 2 μM and 50 μM, may be added to the suspension of cells. In another embodiment, an efficient concentration between 0.05 μM and 2 mM of matrix metalloproteinase inhibitors (e.g. GM6001), more preferably between 2 μM and 100 μM, may be added to the suspension of cells.

In one specific embodiment, an efficient concentration of one or more megakaryocyte modulator compound is comprised in the perfusion medium, which perfusion medium is the medium added for resuspending the cells after the culture and washing steps.

In another embodiment, an efficient concentration of one or more megakaryocyte modulator compound is added after the re-suspension step, within the perfusion medium containing the re-suspended cells, but prior to introducing said suspension of cells in the fluidic device. Alternatively, one or more megakaryocyte modulator compound is added directly in the fluidic device, during the perfusion stage of the cells.

In particular, in one specific embodiment, an efficient concentration of said megakaryocyte modulator compound is added only to the suspension of cells after the washing step and is not present during the culture stage. Alternatively, in another specific embodiment, said megakaryocyte modulator compound is added in an

efficient concentration for polyploidization and proplatelet formation during the culture stage, and is further added a second time after the washing step (to be present during the perfusion stage) at an efficient concentration for increasing the number of platelets released per megakaryocyte circulating in the channel of the fluidic device.

As used herein, the term "megakaryocyte modulator" thus relates to any compound that is known to increase directly or indirectly megakaryocyte ploidy and/or is involved in cytoskeleton organization.

As used herein, the term "megakaryocyte ploidy" refers to the number N of sets of chromosomes in the said megakaryocyte. Ploidy increase occurs through endomitosis in which a dividing megakaryocyte arrests during anaphase B and the cleavage furrow degenerates, resulting in a polyploid cell with double the previous DNA content. Increase in

megakaryocytic ploidy is associated with increase in megakaryocytic volume, a large size and an abundant cytoplasm (Ravid et al, J Cell physiology, Vol. 190, 7-20, 2002). An increase of megakaryocyte ploidy under culture conditions can be visualized for example by a flow cytometry analysis method as described in Avanzi et al {British Journal of Haematology, Vol. 164, 867-876, 2014).

As used herein, compounds involved in cytoskeleton organization refers to any compound that has a visible effect on the cytoskeleton organization of an eukaryotic cell, for example such visible effect can be the reorganization (e.g. polymerization and/or depolymerization) of actin filaments, myosin filaments, microtubules through for example the dynein/dynactin and kinesin motors.

According to the invention, said megakaryocyte modulator compound is selected from:

i. an inhibitor of the Rho/ROCK pathway capable of increasing megakaryocytes ploidy,

ii. nicotinamide,

iii. blebbistatin,

iv. latrunculin, and

v. GM6001.

These seven compound families are described in further details below:

***Inhibitor of the Rho/ROCK pathway***

Compounds known to increase megakaryocyte ploidy and which may be used as megakaryocyte modulator compound include Rho/ROCK inhibitors.

The terms "Rho/ROCK inhibitors" or "inhibitors of the Rho/ROCK pathway" as used herein relate to compounds capable of fully or partially preventing, or reducing or inhibiting Rho/ROCK signalling pathway.

As used herein the term "signalling pathway" or "signalling activity" refers to a biochemical causal relationship generally initiated by a protein-protein interaction such as binding of a growth factor to a receptor, resulting in transmission of a signal from one portion of a cell to another portion of a cell. In general, the transmission involves specific phosphorylation of one or more tyrosine, serine, or threonine residues on one or more proteins in the series of reactions causing signal transduction. Penultimate processes typically include nuclear events, resulting in a change in gene expression.

In specific embodiments, the term "Rho/ROCK inhibitor" is intended to refer to a substance that reduces, decreases and/or inhibits Rho/ROCK signalling activity as measured for example by the relative amount of phosphorylated substrate proteins of ROCK kinase.

In a specific embodiment, said Rho/ROCK inhibitors are compound inhibitors inhibiting ROCK kinase activity. Specific inhibition may be measured as IC50 in a functional assay for ROCK kinase activity and the selected inhibitors may have an IC50 of 100μM or less, 10μM or less, 1μM or less, 100nM or less, 10nM or less or 1nM or less.

Methods such as immunoassays and kits for detecting and measuring relative amount of phosphorylated substrate proteins of ROCK enzyme are commercially available (see for example, Merck Millipore, CSA001 / Rho-associated Kinase (ROCK) Activity Assay; CellBiolabs, INC. / STA-416 / Rho kinase (ROCK) Activity Assay, 96 Wells; CellBiolabs, INC. / STA-415 / Rho kinase (ROCK) Activity Immunoblot Kit).

Such inhibitors may thus be selected among small molecule, siRNA, shRNA, anti-sense DNA and the like.

An example of Rho-Rock inhibitor is fasudil (CAS 103745-39-7, or 5-(1,4-Diazepane-1-sulfonyl)isoquinoline)) with the following formula,

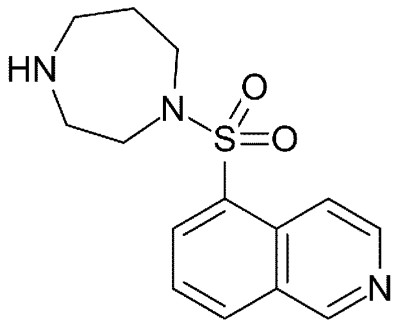

or, more preferably, its active metabolite, hydroxyfasudil (also termed "Y27632"; CAS 155558-32-0; 1-(1-Hydroxy-5-isoquinolinesulfonyl)homopiperazine)), both being available commercially. Another example is thiazovivin which compound is disclosed in Xu et al (PNAS, Vol. 107(18), 8129-8134, 2010). Other Rho-Rock inhibitors which may be used as megakaryocyte modulator compounds have been described for example, without limitation, in US8093266, US8541449, US8604218, US8697911, US8796458, US9000154.

Rho-Rock inhibitors added to megakaryocyte culture showed a dramatic increase of the percentage of high ploïdy cells. Rho-Rock inhibitors inhibit the Rho-Rock pathway, and consequently inhibits myosin activation (through the inhibition of MLC2 (Myosin Like Chain II) phosphorylation) and the contractile ring formation This presumably forces the megakaryocyte to undergo polyploidization (Lordier et al, Blood, Vol.112(8), 3164-3174, 2008), and then proplatelet formation (Chang et al, Blood, Vol. 109, n° 10, 4229-4236, 2007; Avanzi et al, British Journal of Haematology, Vol. 164, 867-876, 2014).

C3 transferase, a direct RhoA inhibitor, or Rho inhibitor Tat-C3 compound may also be used as RhoA/ROCK inhibitor (Shi et al, The Journal of Clinical Investigation, Vol. 124, n° 9, 3757-3766, 2014; Chang et al, Blood, Vol. 109, n° 10, 4229-4236, 2007).

***Inhibitor of the MLCK pathway***

MLC2 phosphorylation is mainly regulated by two different kinases: ROCK and MLCK (Myosin Light Chain Kinase).

Therefore, in the presence of a MLCK inhibitor, MLC2 phosphorylation is also inhibited and proplatelet formation is increased.

In specific embodiments, the term "MLCK inhibitor" is intended to refer to a substance that reduces, decreases and/or inhibits MLCK signalling activity as measured for example by the relative amount of phosphorylated MLC2 protein.

In a specific embodiment, said MLCK inhibitors are compound inhibitors inhibiting MLCK kinase activity. Specific inhibition may be measured as IC50 in a functional assay for MLCK kinase activity and the selected inhibitors may have an IC50 of 100μM or less, 10μM or less, 1μM or less, 100nM or less, 10nM or less or 1nM or less.

Methods such as immunoassays and kits for detecting and measuring relative amount of phosphorylated MLC proteins have been described for example in Egot et al (Thrombosis and Haemostasis, Vol. 110, 1215-1222, 2013).

Such inhibitors may thus be selected among small molecule, siRNA, shRNA, anti-sense DNA and the like.

Examples of MLCK inhibitor include without limitation the compound P18 as disclosed in Lukas et al, Journal of Medicinal Chemistry, Vol. 42, n° 5, 910-919, 1999, and the compound ML-7 as disclosed in Egot et al, Thrombosis and Haemostasis, Vol. 110, 1215-1222, 2013.

***Inhibitor of the sirtuin pathway***

Other compounds known to increase megakaryocyte ploidy and which may be used as megakaryocyte modulator compound include sirtuin inhibitors, such as nicotinamide shown to have an effect on megakaryocyte ploidy (Avanzi et al, British Journal of Haematology, Vol. 164, 867-876, 2014).

The terms "sirtuin inhibitors" or "inhibitors of the sirtuin pathway" as used herein relate to compounds capable of fully or partially preventing, or reducing or inhibiting sirtuin signalling pathway, for example SIRT1, SIRT2 or SIRT3 signalling.

In specific embodiments, the term "sirtuin inhibitor" is intended to refer to a substance that reduces, decreases and/or inhibits SIRT1, SIRT2 or SIRT3 signalling activity as measured for example by the relative amount of acetylated (or deacetylated) substrate proteins of SIRT1, SIRT2 or SIRT3.

In a specific embodiment, said sirtuin inhibitors are compound inhibitor inhibiting SIRT1, SIRT2 or SIRT3 deacetylase activity. Specific inhibition may be measured as IC50 in a functional assay for SIRT1/2/3 deacetylase activity and the selected

inhibitors may have an IC50 of 100μM or less, 10μM or less, 1μM or less, 100nM or less, 10nM or less or 1nM or less.

Methods such as immunoassays and kits for detecting and measuring relative amount of phosphorylated substrate proteins of SIRT1, SIRT2 or SIRT3 enzymes are commercially available (see for example, SIGMA, CS1040 / SIRT1 Assay Kit; Abcam, ab156065 / SIRT1 Activity Assay Kit; Abcam, ab156066 / SIRT2 Activity Assay Kit; Abcam, ab156067 / SIRT3 Activity Assay Kit). Such inhibitors may thus be selected among small molecule, siRNA, shRNA, anti-sense DNA and the like.

An example of sirtuin inhibitor is the nicotinamide (NIC or pyridine-3-carboxamide), one form of niacin (vitamin B3) (Giammona et al, British Journal of Haematology, Vol. 135, 554-566, 2006), with the following formula:

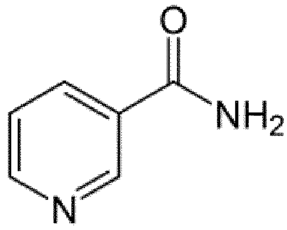

One of the cell functions of nicotinamide is the inhibition of the activity of the silent information regulator (Sir2) family of histone/protein deacetylases (sirtuins or SIRTs). Giammona et al (Exp Hematol., Vol. 37(11), 1340-1352, 2009) suggested that nicotinamide increases megakaryocyte ploidy by inhibition of SIRT1 and/or SIRT2. This resulted in the increased acetylation of one of the target of SIRT deacetylase: p53. The effect of nicotinamide, as a sirtuin inhibitor, is mediated at least by increased p53 acetylation leading to increased megakaryocyte ploidy.

Other sirtuin inhibitors include for example sirtuin inhibitor IV (CAS 14513-15-6 or 2,3-dihydro-5-[(2-hydroxy-1-naphthalenyl)methyl]-6-phenyl-2-thioxo-4(1H)-pyrimidinone) from Cayman Chemical, or for example, splitomicin, HR73, Sirtinol, AGK2, Cambinol, Salermide, Tenovin, Suramin as described in Villalba et al (Biofactors, Vol. 38(5), 349-359, 2012).

### *Inhibitor of the NMMII ATPase*

Other compounds shown to increase megakaryocyte ploidy and which may be used as megakaryocyte modulator compound include NMM II ATPase inhibitors, such as blebbistatin shown to have an effect on megakaryocyte ploidy (Avanzi et al, British Journal of Haematology, Vol. 164, 867-876, 2014).

The terms "NMM II ATPase inhibitors" or "inhibitors of the NMMII ATPase" as used herein relate to compounds capable of fully or partially preventing, or reducing or inhibiting non-muscle myosin II ATPase activity (NMM II ATPase also known as MYH9 or MYH10).

In one specific embodiment, the term "NMM II ATPase inhibitor" is intended to refer to a substance that reduces, decreases and/or inhibits NMMII ATPase activity as measured for example in Shin et al (PNAS, Vol. 108, n°28, 11458-11463, 2011) by performing immunoblot to detect phosphorylated NMM IIA, or in Limouze et al (J Muscle Res Cell Motil, Vol.25(4-5), 337-341, 2004) measuring the actin-activated MgATPase activity.

Specific inhibition may be measured as IC50 in a functional assay for NMMII ATPase activity and the selected inhibitors may have an IC50 of 100μM or less, 10μM or less, 1μM or less, 100nM or less, 10nM or less or 1nM or less.

Such inhibitors may thus be selected among small molecule, siRNA, shRNA, anti-sense DNA and the like.

An example of such NMM II ATPase inhibitor is the compound blebbistatin (CAS 674289-55-5 or 1-Phenyl-1,2,3,4-tetrahydro-4-hydroxpyrrolo[2,3-b]-7-methylquinolin-4-one) inhibiting the nonmuscle myosin II ATPase (NMM II), involved in cytokinesis, membrane rigidity and contraction of matrix (Shin et al, PNAS, Vol. 108, n° 28, 11458-11463, 2011; Avanzi et al, 2014 see above), with the following formula:

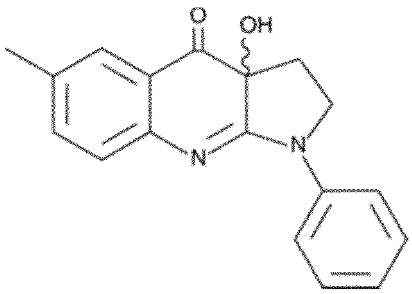

Other inhibitors include for example azidoblebbistatin (Bond et al., Future Med Chem, Vol. 5(1), 41-52, 2013).

***Microtubule organizing regulators***

Microtubule proteins for use as megakaryocyte modulator compounds include proteins directly or indirectly related to the microtubule organization and its functional variants and/or isoforms.

As used herein, microtubule organizing regulators, for use as megakaryocyte modulator compound, refer to proteins involved directly or indirectly in the functional mechanism leading to a dynamic (re)organization of said microtubules proteins, for example involved in polymerization, depolymerization of tubulin as well as microtubule stabilization or sliding.

In one specific embodiment, such microtubule organizing regulators may include microtubule-associated proteins (MAPs), Plus-end tracking protein, motor proteins such as dynein, dynactin or kinesin, proteins involved in tubulin post-translational modifications leading to phosphorylated, ubiquitinated, sumoylated or palmitoylated tubulin, proteins involved in dynamic tubulin instability such as small GTP protein and microtubule polymerases and depolymerases.

Methods such as immunoassays or kits to measuring or detecting any effects of said microtubule organizing regulators, among at least small molecule, siRNA, shRNA, anti-sense DNA and the like, may include, for example, assay from Cytoskeleton, INC., BK038 / Microtubule/Tubulin In Vivo Assay Biochem Kit as well as Cytoskeleton, INC., BK027 / Kinesin Motility Assay Biochem Kit.

***Inhibitor of cytoskeletal signaling***

Compounds known to inhibit cytoskeletal signaling and which may be used as megakaryocyte modulator compound include actin polymerization inhibitor.

The term "inhibition of cytoskeletal signaling" as used herein relates to "inhibitors of cytoskeletal signaling" or "cytoskeletal signaling inhibitors", compounds capable to fully or partially preventing, or reducing, or inhibiting cytoskeletal signaling.

In specific embodiment, the term "inhibitor of cytoskeletal signaling" includes but is not limited to a substance that inhibit actin polymerization, also known to induce apoptosis.

Methods such as immunoassays and kits for detecting and measuring relative amount of non-muscle actin polymerization are commercially available (see for example, Cytoskeleton, cat#BK003 or cat#AP05 combined with cat # APHL99).

Such inhibitors may thus be selected among small molecule, siRNA, shRNA, anti-sense DNA and the like.

An example of actin polymerization inhibitor can refer to the molecule Latrunculin and more specially Latrunculin A, Latrunculia magnifica (CAS 76343-93-6) - Santa Cruz Biotechnologies)), a stabilizer of monomeric G-actin and polymerization inhibitor, with the following formula:

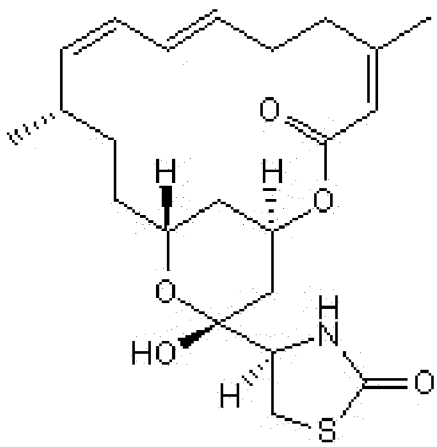

Latrunculin A is described in Avanzi et al, PLoS ONE, 10(4): e0125057 to increase megakaryocyte polyploidization and increase megakaryocyte apoptosis activation.

Other actin polymerization inhibitor can refer to, but is not limited to, the Latrunculin B (CAS 76343-94-7 - Santa Cruz Biotechnology) with the following formula:

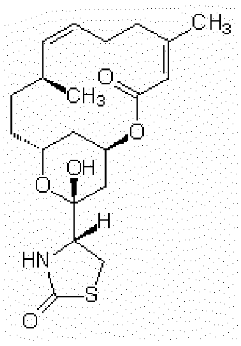

Bistheonellide A (CAS 105304-96-9 - Santa Cruz Biotechnology) with the following formula:

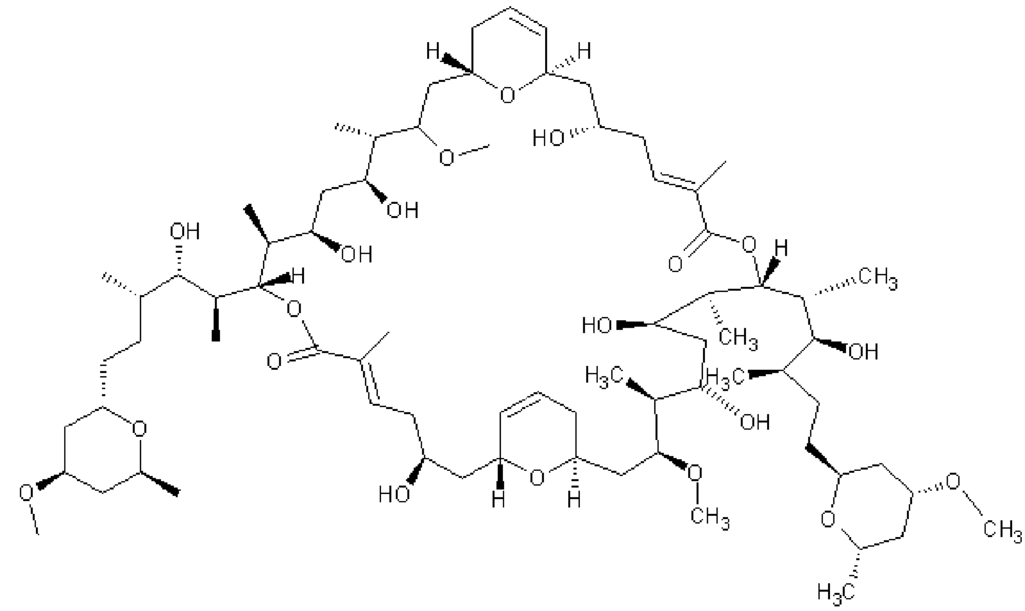

CK 666 (CAT No 3950 - Tocris) with the following formula:

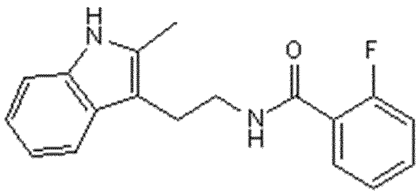

187-1 N-WASP inhibitor (CAT No 2067 - Tocris) with the following sequence cyclo(Lys-D-Phe-D-Pro-D-Phe-Phe-D-Pro-Gln)

CK 869 (CAT No 4984 - Tocris) with the following formula:

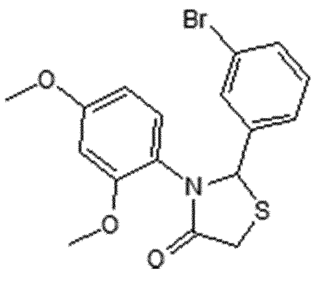

Narciclasine (CAT No 3715 - Tocris) with the following formula:

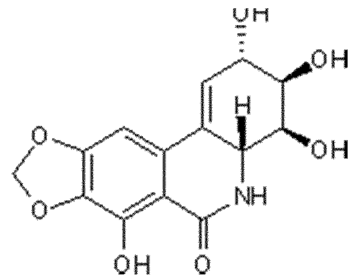

Cytochalasin B (or A to J) (CAT No 5474 - Tocris) with the following formula:

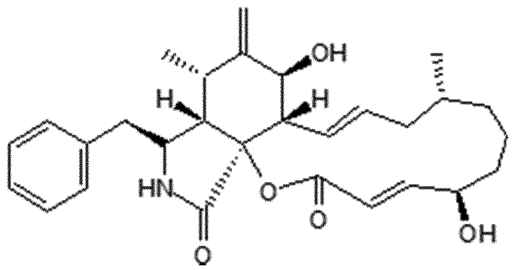

***Protease inhibitor***

Compounds known to inhibit protease include matrix metalloproteinase (MMP) inhibitor.

The term "protease inhibitor" as used herein relates to "inhibitors of protease" or "protease inhibitors", compounds capable to fully or partially preventing, or reducing, or inhibiting protease.

In specific embodiment, the term "inhibitor of protease" includes but is not limited to a substance that inhibits MMP.

Such inhibitors may thus be selected among small molecule, siRNA, shRNA, anti-sense DNA and the like.

An example of MMP inhibitor can refer to the molecule: a pan MMP inhibitor GM6001 (ab120845 - Abcam), also known as Ilomastat (Galardin) or N-[(2R)-2-(hydroxamidocarbonylmethyl)-4-methylpentanoyl]-L-tryptophan methylamide; and which controls the biological activity of MMP, with the following formula,

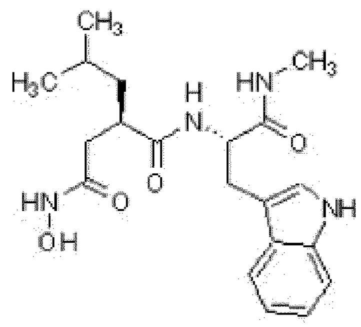

The protease inhibitor may be two or more protease inhibitors and examples of protease inhibitors may be a MMP specific inhibitor such as MMP8-I ((3R)-(+)-[2-(4- Methoxybenzenesulfonyl)-1,2,3,4-tetrahydroisoquinoline-3-hydroxamate, MMP-3 inhibitor, MMP-12 inhibitor, MMP2 inhibitor, MMP9 inhibitor, MMP 13 inhibitor or a broad spectrum MMP inhibitor such as Batimastat, CTS-1027, Marimastat, NSC- 405020, SB-3CT or GM1489.

The use of a protease inhibitor, such as a pan MMP inhibitor (for example GM6001) is particularly advantageous of in an ex vivo method according to the invention for producing platelets expressing CD42b receptor (CD42b$^+$ platelets).

**The fluidic device**

In the following, a fluidic device which can be used to implement the method of the invention will be described as a comparative example.

The fluidic device comprises one production chamber comprising at least one channel having at least two openings, which may be further described as the inlet and the outlet of the channel. The channel is such that a suspension of megakaryocytes or their fragments may flow from its inlet to its outlet. Preferably, the channel has one inlet and one outlet and a main flow can be applied for the suspension of megakaryocytes to flow from said inlet to said outlet. Such fluidic device may be used in the method of the invention for producing platelet.

In specific examples, the production chamber may be further divided into several parallel channels, each of them delimited by non-porous walls, with at least one inlet opening at one end of the chamber in which a suspension of cells comprising megakaryocytes can be introduced and at least one outlet opening at the other end in which platelets can be collected, said channel forming a single flow from its inlet to its outlet.

As used herein, the term "non-porous" means that the megakaryocytes or their fragments (including platelets or proplatelets) cannot cross the walls and thereby remain in the main flow from the inlet to the outlet of the channel.

In order to produce platelets with a high yield, the channel of the fluidic device may be textured on at least one portion of its inner surface, i.e. on at least one portion of the surface of the channel's wall, which is intended to be in contact with the suspension of megakaryocytes. The texture is generated by a plurality of obstacles. Preferably, said obstacles are distributed on at least one 2-dimensional surface of the channel, thereby forming a 3-dimensional array of obstacles. Because of said obstacles, the inner surface of the channel is not smooth. The array of obstacles may be organized in a periodic manner.

In one specific example, the density of the obstacles may be constant along the channel. When flowing through a textured channel, the capture of megakaryocytes on the textured surface and their shedding into platelets are improved.

The inventors further discovered that a variation of the density of obstacles can reduce the 'clogging' of cells, and have an impact on the capture of megakaryocytes. The platelets that are generated by exposure of megakaryocytes to the flow through the textured channel display some functional aspects resembling those of the circulating blood platelets. In particular, in specific examples, the platelets produced in the device of the present invention can be activated, like natural platelets or circulating blood platelets, in contrast to platelet-like particles, which cannot be activated.

According to a preferred example, at least a portion, optionally a textured portion, of the inner surface of the channel may be further coated with a ligand with binding affinity for megakaryocytes, e.g., von Willebrand factor (VWF) or its functional variants, or fibrinogen or fibronectin. Such ligand may have specific affinity for platelets and/or megakaryocytes or their specific receptors, or, non-specific affinity for platelets and/or megakaryocytes. Such coating increases cell adhesion to the inner wall or obstacles of the channel.

As used herein, the term "von Willebrand factor" or "VWF" denotes the multimeric protein consisting of several monomers involved in hemostasis. An exemplary amino acid sequence of human von Willebrand factor can be found in the GenPept database

under accession number AAB59458. Preferably the von Willebrand factor according to the invention is a mammalian Von Willebrand factor, even more preferably a murine factor or a primate factor, even more preferably human von Willebrand factor.

The term "VWF" encompasses VWF of any mammalian origin, such as primate VWF, preferably human VWF. According to the invention, the VWF factor can be recombinant VWF or native VWF. In its native form, it can be purified or can be comprised in a composition comprising other components (e.g. in an extracellular matrix). The skilled person can also easily produce such recombinant VWF according to standard techniques in the art, for example using transfected host cells capable of producing said recombinant VWF or its functional variants.

As used herein, the term "functional variant" of VWF, refers to natural or recombinant fragment of VWF factor, or homologue or analogue of VWF retaining the capacity to bind to GPIb, especially human GPIb.

A homologue of VWF is a polypeptide with an amino acid sequence, which shares at least 50% identity, preferably, at least 60%, 70%, 80%, 90%, and 95% identity to human VWF amino acid sequence.

As used herein, the percent identity between two sequences is a function of the number of identical positions shared by the sequences (i.e., % identity = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described below.

The percent identity between two amino acid sequences can be determined using the algorithm of E. Myers and W. Miller (Comput. Appl. Biosci. Vol. 4: 11-17, 1988) which has been incorporated into the ALIGN program. In addition, the percent identity between two amino acid sequences can be determined using the Needleman and Wunsch (J. Mol. Biol. Vol. 48, 443-453, 1970) algorithm, which has been incorporated into the GAP program in the GCG software package. Yet another program to determine percent identity is CLUSTAL (M. Larkin et al, Bioinformatics Vol. 23, 2947-2948, 2007; first described by D. Higgins and P. Sharp, Gene, Vol. 73, 237-244, 1988) which is available as stand-alone program or via web servers (see http://www.clustal.org/).

Variants further include polypeptide or fusion protein comprising a fragment of VWF factor fused to a heterologous polypeptide sequence, which is not naturally linked to said VWF. Other variants include multimeric forms of VWF

domains, wherein said VWF domains are those fragments of VWF, which have affinity to GPIb.

As used herein, the term "fragments" include natural or recombinant partial amino acid sequence of at least 5, preferably at least 10 or at least 20 consecutive amino acids of a given polypeptide. Examples of variants of VWF which can be used for coating the device of the present invention include, without limitation, recombinant VWF-A1 (amino acids Q1238- P1471) polypeptides, which can be expressed in Escherichia coli, or recombinant VWF-A1A2A3 (amino acids D1261-G1874) polypeptide, which can be expressed in mammalian cells and purified as previously described in Martin et al (Journal of Thrombosis and Haemostasis, Vol. 5, 1363-1370 2007, doi: 10.111 1/j.1538- 7836.2007.02536.x.)

Alternatively, the skilled person may select analogues of VWF, i.e. a protein or polypeptide which does not share sequence homology with VWF primary amino acid sequence, but exhibit similar properties of megakaryocyte and/or platelet binding affinity. Such analogues may be selected without limitation from the group consisting of fibrinogen, fibronectin, laminin, collagen and tenascin.

The channel of the fluidic device according to the invention may be defined by its length L between its inlet and its outlet. The cross-section of the channel, which may be preferably the same on the whole length of the channel, may be round, ovoid, rectangular or square. As used herein, the height H of the channel refers to the smallest distance measured between two opposite walls in a section of the channel. For example, in a circular section, the height H of the channel is the diameter of the circular section of the channel. According to one preferred example, said channel may have a substantially square or rectangular section, with bottom and upper walls determining the channel height H, and side walls determining the channel width W. The channel is preferably straight and the axis of the straight channel defines the longitudinal direction of the channel.

According to a preferred example, the fluidic device is a microfluidic device, i.e. one of the dimensions of the cross-section of the device is smaller than 1 millimeter. The channel may be called microchannel.

With regards to the dimension of the channel, these may be optimized depending on the average size of the megakaryocytes that could be used for platelet production. Such parameter called $D_{cell}$ throughout the specification is defined as the mean megakaryocyte diameter in the suspension for use in the method of the present invention, and can be measured by different techniques, as impedance cell counter detection (for example a Coulter counter as described in US Patent 2,656,508) or optical microscopy followed by image analysis.

Depending on the source of precursor cells and the megakaryocyte production method that are used, $D_{cell}$ may typically be comprised between 5 $\mu$m and 150 $\mu$m, for example between 7 $\mu$m and 100 $\mu$m, and for example between 10 and 50 $\mu$m. In specific examples, the dimensions of the device of the invention are optimized for $D_{cell}$ being selected among the following sizes: 15 $\mu$m, 20 $\mu$m, 25 $\mu$m, 30 $\mu$m, 35 $\mu$m, 40 $\mu$m and 45 $\mu$m.

In specific examples, the height H of the channel may be comprised between $D_{cell}$ and 1 mm, preferably between $D_{cell}$ and 100 $\mu$m. The width W of the channel may be comprised between $D_{cell}$ and 1 m, preferably between 10x $D_{cell}$ and 1 cm. The length L of the channel may be comprised between 1 mm and 10 m, preferably between 10 mm and 1 m, and more preferably between 10 cm and 1 m.

The production chamber of the fluidic channel of the invention may comprise one single channel or a plurality of channels, which may be different or identical. When a plurality of channels is used, parallelized channels share the same flow inlet and flow outlet. According to a preferred examples, the production chamber may comprise a plurality of parallelized channels. The number N of channels in the production chamber may be comprised between 2 and 1,000,000, preferably between 2 and 100,000.

When parallelized channels are used, the production chamber preferably further comprises a distribution channel, which distributes the inlet suspension to every channel. The distribution channel may have a triangular shape. Advantageously, the
shape of the distribution channel is such that it avoids damaging the cells with a too high shear rate, for instance by having a higher height than the one used in the parallelized channels.

The inner surface of the channel is textured on at least one portion. Said textured portion of the channel may have a length comprised between 1 % of the length L of the channel and 100% of the length of the length L of the channel, preferably, between 50% of the length L of the channel and 100% of the length L of the channel.

The texture can be generated by a plurality of obstacles. The density, size and shape of said obstacles may be determined so as to capture megakaryocytes on the surface of obstacles and/or of the inner channel walls for further platelet shedding.

As used herein, the term "capture" means that the megakaryocytes contact the surface of obstacles and/or the inner channel and are strongly slowed down, as compared to the mean velocity of the flow. The megakaryocytes can be possibly stopped after contacting the surface of an obstacle. The obstacles may have for instance the shape of posts, pillars, beams, crescent, pierced-crescent, stars, cavities or pyramids. Preferably, the obstacles are posts. A post may preferably have a square or round or triangle cross-section, and it can be defined by its radius r and its height h. As used therein, the "radius" of the post is defined as the half of the largest dimension of the cross-section of the post. With regards to the dimension of the posts: the height h of the posts may preferably be comprised between 0 and H. The radius r of the posts may preferably be comprised between $D_{cell}$/100 and 100x $D_{cell}$, more preferably between $D_{cell}$/10 and 10x $D_{cell}$. $D_{cell}$

being often comprised between 5 and 150 μm, the height h of the posts may preferably be comprised between 0 and 1 mm. The radius r of the posts may preferably be comprised between 50 nm and 15 mm, more preferably between 500 nm and 1.5 mm.

The obstacles may be placed on the inner surface of the channel randomly or according to a specific pattern. Consequently, the texture may be irregular or regular. A regular pattern may be characterized by some features like its periodic structure, the lattice pitch and the tilt of the lattice direction with regard to the longitudinal direction of the channel. If the pattern is irregular, it may be characterized by the density of the
obstacles or by the mean distance between obstacles in the longitudinal and in the transversal direction.

In one example, the obstacles may be arranged on the inner surface of at least one portion of the channel to form a regular pattern. According to another example, the obstacle density may vary along the channel.

For example, said channel may be textured with a plurality of obstacles wherein the longitudinal and/or transversal distances between two obstacles decrease with respect to the flow direction (from the inlet to the outlet of the channel) to result in an increasing density of the obstacles.

The density of obstacles can be measured as the number of obstacles per surface area of the textured portion of a channel.

In a more specific example, said obstacles can be arranged on the inner surface of at least one portion of the channel and form a hexagonal periodic structure, wherein the density of obstacles increases by reduction of the closest distance between each obstacle in the flow direction.

According to one specific example, the obstacles are posts with a substantially circular section of a radius r, and said posts are arranged on the inner surface of at least one portion of said channel to form a regular pattern with a hexagonal periodic structure such as:

(i) the closest distances p and g between two post centers respectively along the longitudinal and the transversal axes are at least equal to 2r, preferably between ($2r + D_{cell}/10$) and ($2r + 100xD_{cell}$), even better between ($2r + D_{cell}$) and ($2r + 10xD_{cell}$);

(ii) the distance p between two post centers along the transversal axis varies along the channel to increase and/or the distance g between two post centers along the longitudinal axis varies along channel, in order to increase the obstacle density in the flow direction, namely from the inlet of the channel to the outlet of the channel;

(iv) the angle α, which is the lowest angle defined by the longitudinal direction of the channel and one of the lattice vectors of the primitive cell of the hexagonal periodic structure, is such as: 2

- when $r \geq D_{cell}/2$, α is comprised between $\arcsin(\frac{r}{10g})$ and $\arcsin(\frac{2r}{g})$, ,
- when $r < D_{cell}/2$, α is comprised between $\arcsin(\frac{Dcell}{20g})$ and $\arcsin(\frac{Dcell}{g})$

(iv) optionally said posts have a height h such as $0 < h \leq H$, wherein H is the height of the channel, for example h=H.

In one specific example, the obstacles are posts with a substantially circular section of a radius r, and said posts are arranged on the inner surface of at least one portion of said channel to form a regular pattern with a hexagonal periodic structure such as:

(i) the radius of a post r is preferably between 50 nm and 15 mm, more preferably between 500 nm and 1.5 mm;
(ii) the closest distances p between two post centers along the transversal axis and the closest distance g between two post centers along the longitudinal axis are at least equal to 100 nm, preferably between 100 nm and 50 mm, preferably between 500 nm and 10 mm, even better between 5 μm and 1 mm;
(iii) the angle α, which is the smallest angle defined by the longitudinal direction of the channel and one of the lattice vectors of the hexagonal Bravais lattice is such as a is comprised between 0 and 90° and preferably between 0 and 30°;
(iv) optionally said posts have a height h such as $0 < h \leq H$, wherein H refers to the smallest distance measured between two opposite walls in a section of the channel, for example h=H.

The fluidic device may preferably be made according to commonly known techniques of fabrication of microfluidic system, for instance by soft lithography (Xia et al, Annual Review of Materials Science, Vol. 28, n°l, 153-184, 1998).

A master of the device with positive relief of the at least partly textured channel may be prepared by conventional

methods: one method may consist in producing transparencies from a computer assisted design file containing the design of the channel. After that, these transparencies may be used as masks in transferring the pattern into a negative photoresist to form the master.

The fluidic device may be made of polydimethylsiloxane (PDMS) and sealed on glass slides. This material is advantageous for having a visual control of the device. However, other materials like silicon, glass, polystyrene, polycarbonate, polyvinyl chloride, cyclic olefin copolymer, poly(methyl methacrylate), thermoset polyester, polyurethane methacrylate, Norland Optical Adhesive, hydrogels (e.g. alginate, collagen, agarose, polyacrylamide, polysaccharide) can be used for the fabrication of the fluidic device.

Advantageously, the fluidic device and more specifically the production chamber allows to work under sterile conditions. In a preferred example, said fluidic device is sterile. The method for producing the fluidic device according to the invention may comprise the step of sterilization of the device. This sterilization step may occur before or after the sealing of the device.

In addition, the production method of the fluidic device may comprise the step of coating at least the textured portion of the inner surface of the channel with a ligand with binding affinity for megakaryocytes, e.g., von Willebrand factor (VWF) or its functional variants. In a particular example, the inner surface of the channel is coated by incubation with a solution of von Willebrand factor or its functional variants. Typically, the concentration of VWF used for coating the solid phase is between 5 and 100 $\mu$g/mL. In a preferred example, the concentration of VWF is between 20 and 40 $\mu$g/mL. In one example, the inner surface of the channel is coated with functional variants of VWF selected from the group consisting of recombinant wild-type VWF or mutated VWF polypeptides, expressed in E. coli or in mammalian cells, as monomeric or dimeric polypeptides.

In addition to a production chamber, the fluidic device according to the invention may comprise optional means such as:

- a flow rate controller for controlling the flow of said suspension in the channel,
- optionally a megakaryocyte sorter and/or a mixer for enriching the suspension with megakaryocytes and homogenizing cell concentration of said suspension of megakaryocytes, upstream of the production chamber,
- optionally a platelet sorter downstream from the production chamber for purifying the outflow suspension by sorting platelets from megakaryocytes or other cell residues.

The flow rate controller may be placed on the channel so as to control the flow rate preferably at the inlet of the channel or at the outlet of the channel. This means may optionally be coupled with a pump, which generates the flow of the suspension of megakaryocytes into a fluidic device.

The use of a megakaryocyte sorter upstream of the production chamber may be advantageous to obtain a suspension homogeneous in terms of cell population and to obtain consistent yield and quality for industrial production of platelets. In particular, the megakaryocyte sorter includes means to separate the megakaryocytes from platelets and other cell residues. Conventional cell sorter as described below for the platelet cell sorter may be used. In particular, the use of a megakaryocyte sorter allows to obtain a cell suspension enriched in megakaryocytes, i.e. wherein at least 50%, preferably at least 70%, 80%, 95% or about 100% of the cells in the suspension are megakaryocytes.

The use of a mixer may further be advantageous for ensuring a good homogeneity of the suspension, which is entering into the production chamber of the fluidic device, in particular if the production chamber comprises several channels. Cell mixer may be for instance of the type disclosed by Stroock et al. (Science, Vol. 295, n°5555, 647-651, 2002).

At the outlet of the production chamber, the outflow contains produced platelets but it may further contain naked nuclei and/or intact megakaryocytes. A means for separating the produced platelets may be thus advantageously put downstream of the production chamber. Conventional cell sorter device may be used, such as those fitted for microfluidic techniques or for large volumes like apheresis techniques. The platelet sorter may be selected from the group consisting of a cross-flow filtration device, a laminar-flow-based cell sorter, a dielectrophoresis-activated cell sorter, an optical force-based cell sorter, a magnetic force-based cell sorter, and acoustic force- based cell sorter and an inertial forces-based cell sorter. Among the laminar-flow- based cell sorters, the device may be a cell sorter based on the pinched-flow fractionation technique, as disclosed for example by Takagi et al. (Lab Chip, Vol. 5, n°7, 778, 2005) or based on the Deterministic Lateral Displacement, as described for example by L. R. Huang et al. (Science, Vol. 304, 987, 2004).

**The perfusion stage**

The invention is directed to an ex vivo method for producing platelets from a suspension of cells comprising megakaryocytes or their fragments. Said method is carried out by perfusing the suspension of cells, supplemented with at least one megakaryocyte modulator compound, into a fluidic device according to the invention as disclosed in the above

section. All particular features and embodiments disclosed above for the fluidic device are thus features and embodiments of the method.

First, the method comprises the step of introducing a suspension of megakaryocytes or their fragments into the fluidic device. The perfusion medium comprising the cells includes an efficient concentration of at least one megakaryocyte modulator compound, for example, a Rho/Rock inhibitor, an inhibitor of the MLCK pathway, an inhibitor of the sirtuin pathway, an inhibitor of NMM II ATPase, a microtubule organizing regulator, an inhibitor of cytoskeletal signaling and/or an inhibitor of the matrix metalloproteinase The flow of said suspension may be generated and controlled by a pump optionally coupled with a flow rate controller. Typically, each channel of the production chamber defines a single flow, allowing the suspension of megakaryocytes or their fragments to flow from its inlet to its outlet through the production chamber. Accordingly, the flow is directed from the channel inlet to the channel outlet. According to a preferred embodiment, the flow introduced into the fluidic device is continuous.

As disclosed above, the suspension of megakaryocytes may be a suspension obtained from cells isolated from a donor or obtained by differentiation of progenitor cells, for example progenitor cells selected from the group consisting of hematopoietic stem cells, embryonic stem cells and induced pluripotent stem cells.

In the fluidic device, the suspension of megakaryocytes or their fragments is brought to the production chamber. According to a preferred embodiment, prior to entering in the production chamber said suspension is sorted using the cell sorter and/or homogenized. The homogenization may be carried out in a mixer upstream from the production chamber. Then, the suspension of megakaryocytes or their fragments is brought at the inlet of one or more channels in the production chamber.

According to another specific embodiment, said suspension of megakaryocyte include megakaryocytes which have been subjected to a first passage in the fluidic device and are thus reintroduced at the inlet of the channel, optionally, supplemented with at least one megakaryocyte modulator compound.

After its introduction, the suspension of megakaryocytes or their fragments is subjected to a flow under shear rate suitable for elongation, fragmentation of the megakaryocytes and platelet release in the channel of the production chamber. The skilled person will be able to select appropriate shear rate.

For example, in specific embodiments, preferably with channels with no obstacles (i.e. no textured portion of the channel), the shear rate within the channel is at least 300 $s^{-1}$, preferably at least 600 $s^{-1}$, and more preferably at least 1000 $s^{-1}$, for example between 300 $s^{-1}$ and 8000 $s^{-1}$, or between 600 $s^{-1}$ and 2400 $s^{-1}$.

As used herein, the term "wall shear rate" ($\dot{\gamma}_w$) refers to the parameter used to characterize the interaction of the flow with the surface of the channel, the obstacles or the megakaryocytes.

The wall shear rate $\dot{\gamma}_w$ is defined at any local surface element. On a specific surface element can be defined a normal vector $\hat{n}$ and a vector $\hat{m}$ tangential to the surface and in the local direction of the fluid velocity $\hat{v}$ so as ($\hat{n}$, $\hat{m}$) is a planar Cartesian coordinate system. The wall shear rate is then defined by $\frac{\partial\, \hat{v}.\hat{m}}{\partial n}$. The SI unit of measurement for shear rates is $s^{-1}$.

In specific examples where the channel of the fluidic devices includes obstacles to improve capturing of the megakaryocytes (i.e. a textured portion of the channel), the value of the wall shear rate will change locally because of the size, shape and position of the obstacles. Consequently, in a textured portion of the channel of the fluidic device, the suspension of megakaryocytes or their fragments is subjected to a flow under a shear rate $\dot{\gamma}$; which may vary between a minimum value $\dot{\gamma}_{min}$ and a maximum value $\dot{\gamma}_{min}$.

In such specific examples, the flow rate may be fixed within a range value enabling to subject said megakaryocytes in the textured portion of the channel to a wall shear rate $\dot{\gamma}_w$ ranging from 0 to a maximum value $\dot{\gamma}_{min}$, preferably not exceeding 30000 $s^{-1}$, preferably 10000 $s^{-1}$, more preferably 8000 $s^{-1}$ and even more preferably 5000 $s^{-1}$.

The shear rate $\dot{\gamma}_w$ a textured portion of the channel of the device according to the invention may be numerically calculated using the finite element method, for a given channel geometry, suspension liquid phase parameters and flow rate.

By comparison, in the human circulation, the wall shear rates vary from 30-40 $s^{-1}$ in the largest veins to 5000 $s^{-1}$ in the microcirculation.

At the outlet of the channel, the collected suspension contains produced platelets. According to an aspect of the invention, the collected suspension at the outlet of the channel may further contain naked nuclei and/or intact megakaryocytes.

Consequently, the method may further comprise a step of purifying, enriching or separating platelets from said suspension. In addition, said platelets may be sorted out at the outlet of the channel. The sorting may be carried out with a cell sorter downstream of the production chamber, as disclosed above. For instance, the sorting may be carried out by a method selected from the group consisting of cross-flow filtration, laminar flow-based sorting, dielectrophoresis-based sorting, optical force- based sorting, magnetic force-based sorting, acoustic force-based sorting or inertial forces-based sorting.

Either sorted intact megakaryocytes (which have not been elongated at the first passage), or, the whole

suspension of cells, may be collected at the outlet of the channel and reintroduced in the fluidic device for one or more further passages within the channel for further platelet release. Indeed, it may be advantageous to submit the suspension of cells to more than one passage within the channels to optimize platelet production.

Alternatively, either sorted intact megakaryocyte, or, the whole suspension of cells, may be collected at the outlet of the channel and cultured for further maturation into megakaryocyte, prior to reintroducing as a suspension of cells in the fluidic device for further platelet production.

**Platelets obtainable by the method and their use**

Advantageously, the method of the invention enables to produce platelets from a suspension of megakaryocytes with a high yield production while maintaining the functional qualities of the newly generated platelets.

Platelets obtained or obtainable by the described above may be used for the preparation of a pharmaceutical composition, for example for the treatment of decreased platelet count disorders, in particular thrombocytopenia and thrombopathy.

For example, the platelets obtained or obtainable by the method according to the invention may be transfused in an efficient amount to a subject suffering of a disorder of platelet production.

Moreover, the method of the invention for producing platelets is suitable to large-scale production of high quality and standardized platelets. Consequently, produced platelets may be used for diagnostic purposes. They can be used as a normal control for the standardization of platelet function. Indeed, platelet function testing requires fresh blood platelets in native functional condition from normal individuals and affected individuals. Standardization of platelet function testing requires that the laboratory should perform a normal control with every batch of platelet function tests performed. However continuous regular blood sampling by venepuncture raises several health concerns and ethical issues. Advantageously, platelets obtained or

or obtainable by the method of the invention may be used as a positive control in an in vitro diagnostic test for measuring platelet function. More applications can be considered, like the wound healing as well as the development of platelet lysate that has been proposed as acceptable alternatives to foetal calf serum in cell culture, more precisely for mesenchymal stem cells, increasingly used in a wide range of cell therapy.

This invention will be further understood in light of the following non-limiting examples, which are given for illustration purposes only, and also in connection with the attached drawing in which: -

**Figure 1** illustrates the effect of ROCK inhibitor (Ri) added during the perfusion stage in the absence (left panels) or in the presence (right panels) of Ri during the culture stage: (A) Ratio (no Ri vs addition of Ri during the perfusion stage) of means of surface density of captured megakaryocytes (black line) and elongated megakaryocytes (gray line). Means ± SEM is calculated on n=16 channels of the microfluidic device. Statistical analysis was performed using Student t-test (* $p<0.05$, ** $p<0.01$, *** $p<0.001$). The count is made after 35 min of perfusion. Data of one experiment out of 3 are shown. (B) Large-scale images illustrating one embodiment of Ri addition effect in the fluidic suspension during the perfusion stage. Images presented were taken at the position x = 1 cm from the beginning of the textured area after 35 min of perfusion of a megakaryocytes suspension cultured in the absence (left panels) or in the presence (right panels) of Ri; and in the absence (top panel) or the presence (bottom panel) Ri in the fluidic suspension during the perfusion stage. The scale bar represents 100 μm.

- **Figure 2** illustrates the effect of VWF on the cell capture and elongation. (A) Ratio (VWF surface coating vs BSA surface coating) of means of surface density of captured megakaryocytes (black line) and elongated megakaryocytes (gray line). Means + SEM is calculated on n=16 channels of the microfluidic device. Statistical analysis was performed using Student t-test (*** $p<0.001$). The count is made for each experiment after 35 min of perfusion with a megakaryocytes solution containing ROCK inhibitor (Ri) during perfusion stage as well as megakaryocytes culture stage. (B) Typical images of the surface after 35 min of perfusion when using VWF coated microchannels (top panel) and BSA coated microchannels (bottom panel) at the position x = 1 cm from the beginning of the textured area. The scale bar represents 100 μm.

- **Figure 3** is an image illustrating the elongation and fragmentation of a mature megakaryocyte from an inlet solution containing ROCK inhibitor, which does not alter the elongation and fragmentation process. The time montage shows a trapped megakaryocyte with a bead-on-a-thread-like structure undergoing one rupture (black arrow) of the elongated structure. White arrows show the extremity of the elongated megakaryocyte. The scale bar represents 20 μm.

- **Figure 4** is a graph representing the ratio (experimental condition vs twenty-four hours static platelet (STAT 24h) production). Experimental conditions consist in twenty-four hours static platelet production in the presence of ROCK inhibitor (Ri) (STAT 24h Ri), platelet production from megakaryocyte suspension in the absence (+/+ 2h) or in the

presence (+/+ 2h Ri) of Ri, both after two hours of perfusion through the microfluidic system. No Ri was added during the culture stage on the top graph, whereas Ri was added for the bottom panel. Platelet concentrations ([PLP]) are obtained by counting in a hemocytometer. Means ± SEM (top graph: STAT 24h: n=3, STAT 24h Ri: n=2, others conditions: n=6; bottom graph: n=2 for each conditions) of the ratio of the platelet production are provided. Statistical analysis was performed using Student t-test (* $p<0.05$, ** $p<0.01$).

- **Figure 5** is a picture showing two colors flow cytometry analysis of platelets receptors, indicating the population of $CD41^+/CD42b^+$ platelets produced during the perfusion stage from a megakaryocytes suspension cultured (A) in the absence and (B) in the presence of ROCK inhibitor (Ri) during the culture stage. For each panel, experimental conditions consist in twenty-four hours static platelet in the absence (STAT 24h) and in the presence (STAT 24h Ri) of Ri; platelet production during the perfusion stage from megakaryocyte suspension in the absence (+/+ 2h) or in the presence (+/+ 2h Ri) of Ri.

- **Figure 6** is a picture showing single color flow cytometry analysis of platelet receptor density, indicating the number of CD61, CD42b and CD49b receptors on the surface of platelets produced from a megakaryocytes suspension cultured in the absence (top graph) and in the presence (bottom graph) of ROCK inhibitor (Ri) during the culture stage. For each, controls consist in platelets prepared from a blood sample of one donor (black bar), as well as a twenty-four hours static platelet production from megakaryocyte in the absence (STAT 24h) or in the presence (STAT 24h Ri) of Ri. Experimental conditions consist in platelet production during the perfusion stage from megakaryocyte suspension in the absence (+/+ 2h) or in the presence (+/+ 2h Ri) of Ri. n=1 each.

- **Figure 7** is a picture showing microphotographs of adhesion of platelets produced in the microfluidic system from a megakaryocytes solution in the presence (+/+ 2h Ri) or the absence (+/+ 2h) of ROCK inhibitor (Ri) during the two hours of the microfluidic perfusion as well as twenty-four hours static platelet production from megakaryocytes in the absence of Ri (STAT 24h). Indirect immunofluorescence labeling with an anti-$\alpha$-tubulin antibodies, revealed by a secondary AlexaFluor488 anti-mouse antibody and AlexaFluor546 phalloidin for F-actin staining is performed in the absence (left panel) or presence (right panel) of PAR-1 thrombin modulator (TRAP6). Image acquisition was performed with an Axio Observer microscope (Zeiss) at 40x1.6-fold magnification with a QIClick-F-CLR-12 Digital CCD Camera (Q Imaging). Platelets are adherent to fibrinogen.

- **Figure 8** is a picture showing two colors flow cytometry analysis of platelets receptors with or without activation of the CD41/CD61 receptor by the agonist peptide of the PAR-1 thrombin receptor (TRAP6). (A) Histogram indicates, within the platelet gate, the % of PAC-1/CD42b positive elements before (black bars) or after (gray bars) TRAP-6 activation of platelets produced in the microfluidic device from megakaryocytes suspension in the absence (+/+ 2h) or the presence (+/+ 2h Ri) of ROCK inhibitor (Ri). (**B**) displays the corresponding dot plots within the platelet gate, indicating the population of $PAC1^+/CD42b^+$ platelets in the non-stimulated (left panels) and TRAP-6 stimulated samples (right panels) produced in the microfluidic device from megakaryocytes suspension in the absence (+/+ 2h) or the presence (+/+ 2h Ri) of Ri, as well as the control twenty-four hours static platelet production in the presence of Ri (STAT 24h Ri). Notice the shift of the $PAC1^+/CD42b^+$ population in the TRAP-6 stimulated platelets produced in the microfluidic device in the absence or the presence of Ri during the perfusion stage. No Ri was added during the culture stage. n=1.

- **Figure 9** is a picture showing two colors flow cytometry analysis of platelets receptors in the absence or presence of activation of the CD41/CD61 receptor by the agonist peptide of the PAR-1 thrombin receptor (TRAP6). Panels A and B depict data as described in the legend of Figure 8 except that Ri was added during the culture stage. n=1.

- **Figure 10** (**A**) is a graph representing the concentration of platelet produced after 2 hours of perfusion ($[PLP]_{end}$). Experimental conditions consist of platelet production from megakaryocyte suspension in the absence (+/+) or in the presence (+/+ NIC) of nicotinamide, both after two hours of perfusion through the microfluidic system. A control without the microfluidic chip was performed in the absence (+/-) or in the presence (+/- NIC) of nicotinamide during the 2 hours of perfusion. No nicotinamide was added during the culture stage for the 4 first experimental conditions, whereas nicotinamide was added for three last experimental conditions. Platelet concentrations ([PLP]) are obtained by counting in a hemocytometer. Means ± SEM (+/+ and +/+ NIC: n=3; others conditions: n=2) of platelet concentration are provided. (**B - C**) are pictures showing two colors flow cytometry analysis of platelets receptors, indicating the population of $CD41^+/CD42b^+$ platelets produced during the perfusion stage from a megakaryocytes suspension cultured (B) in the absence and (C) in the presence of Nicotinamide during the culture stage. For each panel, experimental conditions consist platelet production during the 2 hours perfusion stage from megakaryocyte suspension in the absence (+/+) or in the presence (+/+ NIC) of Nicotinamide. Additional controls, consisting of 2 hours

perfusion stage without microfluidic chip (+/-) and in the absence (+/-) or in the presence (+/- NIC) during these 2 hours of perfusion, were performed.

- **Figure 11** (**A**) is a graph representing the ratio (experimental condition vs +/+ (without any compound introduction) production). Experimental conditions consist of platelet production ($[PLP]_{end}$) from megakaryocyte suspension in the absence (+/+) or in the presence (+/+ BLEBB) of blebbistatin (BLEBB), and in the presence of DMSO (+/+ DMSO) as a control, during the perfusion, all after two hours of perfusion through the microfluidic system. No blebbistatin was added during the culture stage for the three first experimental conditions, whereas blebbistatin was added during the culture stage for three last experimental conditions. Platelet concentrations ([PLP]) are obtained by counting in a hemocytometer. Means $\pm$ SEM (n=3) are provided. (**B - C**) are pictures showing two colors flow cytometry analysis of platelets receptors, indicating the population of $CD41^+/CD42b^+$ platelets produced during the perfusion stage from megakaryocyte suspension cultured (**B**) in the absence and (**C**) in the presence of blebbistatin. For each panel, experimental conditions consist of platelet production during the 2 hours of perfusion stage from megakaryocyte suspension in the absence (+/+) or in the presence (+/+ BLEBB) of blebbistatin. Additional control, consisting of 2 hours perfusion stage with introduction of DMSO (+/+ DMSO) is provided.

- **Figure 12** (**A**) is a graph representing the ratio (experimental condition vs +/+ (without any compound introduction) production). Experimental conditions consist of platelet production ($[PLP]_{end}$) from megakaryocyte suspension in the absence (+/+) or in the presence (+/+ LA) of latrunculin (LA) during the perfusion, both after two hours of perfusion through the microfluidic system. No latrunculin was added during the culture stage for the two first experimental conditions, whereas latrunculin was added for two last experimental conditions. Platelet concentrations ([PLP]) are obtained by counting in a hemocytometer. n=1 of platelet concentration is provided. Means $\pm$ SEM (+/+ DMSO without LA in cultured MKs n=2; others conditions: n=3) are provided. (**B - C**) are pictures showing two colors flow cytometry analysis of platelets receptors, indicating the population of $CD41^+/CD42b^+$ platelets produced during the perfusion stage from megakaryocyte suspension cultured (**B**) in the absence and (**C**) in the presence of latrunculin. For each panel, experimental conditions consist of platelet production during the 2 hours of perfusion stage from megakaryocyte suspension in the absence (+/+) or in the presence (+/+ LA) of latrunculin during the perfusion.

- **Figure 13** is a picture showing two colors flow cytometry analysis of platelets receptors, indicating the population of $CD41^+/CD42b^+$ platelets produced during the perfusion stage from a megakaryocytes suspension cultured (**A**) in the absence and (**B**) in the presence of GM6001. For each panel, experimental conditions consist of platelet production during the 2 hours perfusion stage from megakaryocyte suspension in the absence (+/+) or in the presence (+/+ GM6001) of GM6001 during the perfusion. Additional control consists of adding DMSO (+/+ DMSO) during the 2 hours perfusion stage.

- **Figure 14** (**A**) is a graph representing the concentration of platelet produced after 2 hours of perfusion ($[PLP]_{end}$). Experimental conditions consist of platelet production from megakaryocyte suspension that was introduced in the microfluidic system at Day maturation (+/+ D12) compared to megakaryocyte suspension that was introduced in the microfluidic perfusion at Day 10 of maturation, then back in culture and re-introduced in the microfluidic system at Day 12 (+/+ D10+D12). Means $\pm$ SEM (n=3) of platelet concentration are provided. (**B**) are pictures showing two colors flow cytometry analysis of platelets receptors, indicating the population of $CD41^+/CD42b^+$ platelets produced during the 2 experimental conditions described above (D12 compared to D10+D12).

- **Figure 15** schematically represents one exemplary geometry of a platelet production with obstacles having a post shape. Figure 10A is a top view and Figure 10B is a cross-section view where r represents the radius of a post, p the closest distance between two post centers along the transversal axis, g the closest distance between two post centers along the longitudinal axis, $\alpha$ is the angle defined by the longitudinal direction of the channel and one of the lattice vectors of the primitive cell of the hexagonal periodic structure, H is the height of the microchannel and h refers to the height of the posts.

- **Figure 16** represents two different gradient geometries used in the example: (A) "gradient geometry 1" and (B) "gradient geometry 2". The numbers inside the device represent the distance p in the corresponding portion of the channel and x the distance from the beginning of the post area.

- **Figure 17** is a stack of images illustrating the effect of the obstacle density on the megakaryocytes capture. The left panel represents a microfluidic device without obstacles density gradient (with p equals to 85 $\mu$m) whereas the right panel represents a microfluidic device with an obstacles density gradient (with p varying from 120 $\mu$m to 70 $\mu$m along the device for the example "gradient geometry 2"). Images from top to bottom are presented at 0, 2.2, 6.6, 8.8 and 13.2

cm from the beginning of the textured area. The images were recorded 35 min after the beginning of the experiment. Scale bar represents 500 μm.

- **Figure 18** is a graph representing the effect of varying the obstacle density on the megakaryocyte capture for two geometry examples. Black triangles correspond to the density σ of captured megakaryocytes in a channel with an increasing post density, while white squares correspond to the density σ of captured megakaryocytes in a channel without obstacles density gradient. The dashed lines indicate the change in
posts density. The texture corresponds to a geometry defined by r = 15 μm, H = 50 μm and h = 50 μm. The device with constant post density is defined by p = g = 85 μm. The upper panel compares this device to the so-called "gradient geometry 1" and defined by g = 85 μm and p varying from 85 μm to 55 μm. The lower panel compares a constant p to the so-called "gradient geometry 2" defined by a varying p from 120 μm to 70 μm. The x axis represents the distance from the textured area entrance. The images were recorded 35 min after the beginning of the experiment.

## EXAMPLES

*Material and methods*

CD34$^+$ cells culture and differentiation

CD34$^+$ cells were isolated from human umbilical cord blood (UCB) by an immunomagnetic technique (Miltenyi Biotec, Paris, France) as previously reported (see Poirault-Chassac et al, "Notch/Delta4 signaling inhibits human megakaryocytic terminal differentiation", Blood, vol.116, n°25, p.5670-5678, 2010). These blood samples were obtained after informed consent and approval from our Institute Ethics Committee and in accordance with the Declaration of Helsinki. CD34$^+$ cells were cultured in a humid atmosphere at 37°C in 5% $CO_2$ in complete medium consisting of Iscove modified Dulbecco medium (IMDM; Gibco Life Technologies, Saint-Aubin, France) supplemented with 15% BIT 9500 serum substitute (Stem Cells Technologies, Grenoble, France), α-monothioglycerol (Sigma-Aldrich, Saint-Quentin Fallavier, France) and liposomes (phosphatidyl-choline, cholesterol and oleic acid; SigmaAldrich). Human recombinant stem cell factor (SCF, 20 ng/mL; Miltenyi Biotec) and thrombopoietin peptide agonist AF13948 (TPO, 50 nM) (see Dunois-Lardé et al, "Exposure of human megakaryocytes to high shear rates accelerates platelet production", Blood, vol.114, n°9, p.1875-1883, 2009) were added once at day 0 to the culture medium followed by addition of 20 nM TPO without SCF at day 5. Mature megakaryocytes are obtained after 12-14 days of culture. Removal of platelets formed during culture and immediately prior to shear exposure was performed by means of a BSA gradient according to the methods reported in (Robert A, Cortin V, Garnier A, Pineault N. Megakaryocyte and platelet production from human cord blood stem cells. Methods Mol Biol. 2012; 788: 219-47). The concentration was then adjusted to 200 000 megakaryocytes / mL. 10 μM ROCK inhibitor (Y27632, Sigma Aldrich, Saint Quentin Fallavier, France) may be added to the culture of megakaryocytes at day 8 of culture. Addition of 10 μM ROCK inhibitor was performed before introducing megakaryocytes suspension into the inlet chamber of the microfluidic device. 6.25 μM nicotinamide (N0636, Sigma Aldrich, Saint Quentin Fallavier, France) was added to the culture of megakaryocytes at day 8 of culture. Addition of 6.25 μM nicotinamide was performed before introducing megakaryocyte suspension into the inlet chamber of the microfluidic device. 20 μM blebbistatin (203390, Calbiochem) was added to the culture of megakaryocytes at day 8 of culture. Addition of 20 μM blebbistatin was performed before introducing megakaryocyte suspension into the inlet chamber of the microfluidic device. 10 μM latrunculin-A (sc202691, Santa Cruz Biotechnology) was added to the culture of megakaryocytes at day 8 of culture. Addition of 10 μM latrunculin-A was performed before introducing megakaryocyte suspension into the inlet chamber of the microfluidic device. 25 μM GM6001 (ab120845, Abcam) was added to the culture of megakaryocytes at day 8 of culture. Addition of 25 μM GM6001 was performed before introducing megakaryocyte suspension into the inlet chamber of the microfluidic device

System architecture

A suspension of mature megakaryocytes is introduced in a 50 mL tube (Falcon tube, Dutscher, France) fixed on an orbital mixer (IKA MS3 basic), rotating at least at 300 rpm. The orbital mixer is used to maintain the homogeneity of the cell concentration in the suspension. The megakaryocytes concentration range in the tube is at least 100 $mL^{-1}$ and cannot exceed $10^{12}$ $mL^{-1}$.

Many methods can be used to control the flow through the different components: a differential pressure controller, a syringe pump and a peristaltic pump for example. When using a peristaltic pump (205U/CA, Watson Marlow, France), both inlet and outlet tubing arrive in the same rotating tube containing the megakaryocyte suspension. The said tube is connected to the inlet of the microfluidic chip with flexible tubing (Tygon ST R-3607, Idex Health and Science, Germany). The suspension is collected at the outlet. The peristaltic pump can be plugged upstream or downstream the microfluidic

components.

Three microfluidic components can be implemented in series: a megakaryocyte sorter and/or a lateral cell mixer upstream, a platelet production channel, and a cell sorter downstream. Cell mixer and cell sorter are optional.

Devices fabrication

Microfluidic components were made following a soft lithography rapid prototyping (Xia et al. 1998. "Soft lithography". Annual Review of Materials Science. vol.28, n°1, p.153-184). First, transparencies were produced from a computer assisted design file containing the design of microchannels. These transparencies were used as masks in transferring the pattern into negative photo resist (SU-8 2000 and 3000 series, Microchem, US) by conventional photolithography, yielding a master with positive relief of micro channels. Both channels were made from molded polydimethylsiloxane (PDMS, Sylgard, Dow Corning, USA), sealed on glass slides. PDMS prepolymer and curing agent were mixed and degassed. The mixture was poured onto the master, cured for 2 h at 70 °C, cut into individual chips, and inlet and outlet holes were punched. Glass slides were cleaned with isopropanol and dried. Both PDMS individual structures and glass slides were treated in an oxygen plasma oven and then sealed.

Platelet production channels

The textured surface is defined by 3D patterns on the channel walls (glass or PDMS). These patterns consist of an array of disks in the (Oxy) plane with a varying density of posts. Herein we present two examples of these possible variations.

The geometries of those patterns are described in **Figure 15** and **Figure 16.** The design of the post array is defined by four parameters: the disk radius r, the distance p between two disk centers along the transverse axis, the distance g between two disk centers along the longitudinal axis and the angle $\alpha$ between the direction of the flow at the inlet and the direction defined by two pillar centers. In the geometries we used for the experiments herein, $\alpha$ was defined by the following criterion: $\sin \alpha = r/g$. This criterion geometrically implies that two neighbor disk centers, once projected on an axis normal to the initial flow direction, are separated by one radius. Experimentally, r varied from 15 $\mu$m to 20 $\mu$m, g varied from 85 $\mu$m to 120 $\mu$m and p varied from 55 $\mu$m to 120 $\mu$m. **Figure 15 (B)** shows the profile of an obstacle in the (Oxz) plane. The height of the channel and the height of the obstacle are defined respectively by H and h. The parameter h/H was experimentally equal to 1 (full pillars).

Two different gradients of p were used in this example, illustrated in **Figure 16 (A)** and **Figure 16 (B):**

- The first one, so-called "gradient geometry 1", was used to increase the megakaryocyte capture in order to increase platelet production. The distance g is fixed at 85 $\mu$m and the distance p is varied from 85 $\mu$m to 55 $\mu$m along the channel. Therefore, the density of posts gradually increases along the channel.

- The second one, so-called "gradient geometry 2", was used in order to homogenize cells density along the device thanks to a starting distance p = 120 $\mu$m. Moreover, this distance p was varied along the channel from 120 $\mu$m to 70 $\mu$m. These variations induce a leap in post density and thus in the megakaryocyte capture. In order to allow enough space for megakaryocyte elongation, the distance g was defined as follows:

  For

$$p \geq 85\ \mu m,\ g = p$$

  For

$$p < 85\ \mu m,\ g = 85\ \mu m$$

All the devices are composed of 16 parallel channels organized in a serpentine shape (**Figure 16**). The channels were textured on the straight parts (13.2 cm long) and patterned with the parameters: r = 15 $\mu$m, H = 50 $\mu$m, H/h = 1.

Shear rates

We define a surface element on the channel wall, whatever on glass or PDMS (including PDMS obstacles). On this surface element, we define the unit vector of a plane by the vector acting normal to it, $\hat{n}$. A unit vector $\hat{m}$, tangential to the surface and in the local direction of the fluid velocity v, is determined so that ($\hat{n}$, $\hat{m}$) is a planar Cartesian coordinate system.

The wall shear rate $\dot{\gamma}$ (in $s^{-1}$) is then defined by $\dot{\gamma} = \frac{\partial\, \hat{v}.\hat{m}}{\partial n}$. The wall shear rate is controlled by both the flow rate in the device and by the geometry of the device.

Videomicroscopy system

The microfluidic chip was set on the stage of an inverted microscope (DMI6000 B, Leica Microsystems GmbH, Germany). A computer assisted motorized stage control was used to record positions along the channel length. We recorded observation field positions and alternated recording images between them along the experiment time. Differential interference contrast objective was used to record movies and images between 10X and 40X. A CMOS high-speed camera (Fastcam SA3, Photron, USA) was used to record images at frequencies from 0.5 to 1500 Hz.

Surface captured cells were counted manually from the recorded channel images, and cells in suspension were counted with a hematocytometer.

Protein surface treatments

Human von Willebrand factor (VWF) was a gift of Laboratoire Français du fractionnement et des Biotechnologies. It was diluted at 40 $\mu g.mL^{-1}$ in phosphate buffered saline phosphate buffered saline (PBS) without calcium and magnesium ions (Lonza, Belgium), and perfused in sealed microchannels. We used this surface coating only in the platelet production channel.

Bovine serum albumin (Sigma-Aldrich La Verpilleres, France) was diluted at 40 $\mu g.mL^{-1}$ in phosphate-buffered saline (PBS) and perfused in microchannels.

For both protein treatments, inlets and outlets of the chips were covered by cover slips. The chips were incubated overnight at 4°C and washed with PBS before the experiment. VWF adsorption on both glass and PDMS was verified by fluorescence labeling with a primary polyclonal rabbit anti-vWF antibody (Dako, 10 $\mu g.mL^{-1}$) and with a secondary Alexa fluor 546 polyclonal goat anti-rabbit antibody.

Parallelization of platelet production channels

A high megakaryocyte flow rate into the device is desired to increase the platelet production number. For a given pattern of obstacles and height of the channel, the flow rate can be increased by increasing the channel width. As mechanical constraints of the PDMS channels impose a maximum width over height ratio to avoid channel collapse, we parallelized channels to increase the effective width.

Megakaryocytes were introduced in the platelet production channel by means of tubing. Cells were distributed in the parallel channel through a triangular shaped entrance, which brings the cells to every channel. For a given channel height, the wall shear rate is inversely proportional to the channel width. Consequently, the wall shear rate is much higher close to the inlet walls than before the entrance of the parallelized channels. To avoid imposing a wall shear rate that could damage the cells, the distribution channel is fabricated using a higher height than the one used in the parallelized shear channels. The ratio of these two heights is typically between 2 and 20.

Platelet sorting

Naked nuclei and intact megakaryocytes can be removed from the platelets by sorting in serial the outflow suspension from the platelet production channel. This can be done with microfluidic techniques. Apheresis techniques can also be considered for large volumes. We give two examples of platelet sorting using the pinched-flow fractionation technique (Takagi et al., Lab Chip, vol.5, n°7, p.778, 2005) and the Deterministic Lateral Displacement (L. R. Huang et al. Science, 304, 987, 2004). The Deterministic Lateral Displacement device is composed of one inlet, an array of posts of spherical shape and two outlets (central and lateral). The device is 5 cm long, 3 mm width and 40 $\mu m$ in height. The post diameter is 85 $\mu m$ and the spaces between posts are 15 $\mu m$. The post row shifting forms an angle of 0.05 radian. The surface is covered by a BSA coating. Cells are deflected and sorted in the central outlet whereas the platelets are not deflected and sorted on the lateral outlets. Another technique to separate platelets from a cells suspension is to use ultrasonic standing wave forces (Antfolk et al, Lab Chip, vol 14, 2791-2799, 2014). By applying an acoustic standing wave field onto a continuously flowing cell suspension, cells can be separated from the surrounding media depending on their physical properties. Then platelets can be sorted.

Characterization of collected platelets

Platelet production in the microfluidic device of geometry 2 was compared to control samples, consisting of

platelet production from twenty-four hours static condition without the microfluidic chip. Expression of CD41 and CD42 antigens was characterized using a flow cytometer BD Fluorescence Accuri C6 ® (BD Biosciences, Le Pont de Claix, France). Platelets were incubated with fluorescein isothiocyanate (FITC)-conjugated anti-human CD41 (αIIb), R-phycoerythrin (PE)-conjugated anti-human CD42b (GPIbα) (both from Beckman Coulter, Villepinte, France) and FITC-conjugated anti-human activated αIIbb3 (BD Biosciences) during 15 minutes at 22°C, Controls were performed using FITC mouse $IgG_1$ (Beckman Coulter), PE mouse $IgG_1$ (Beckman Coulter). Single color flow cytometry analysis of platelet receptors was performed using the GP screen assay (Biocytex, Marseille, France). The number of antigenic sites is determined by converting the fluorescence intensity into corresponding numbers of monoclonal antibodies bound per platelet based on a calibrated bead standard curve. Fibrinogen adhesion assay and epifluorescence characterization were performed as reported in as previously reported in the above-cited publication of Dunois-Lardé, except that activation was obtained in the presence of an agonist peptide of the PAR-1 thrombin receptor (TRAP6). Epifluorescence was analyzed at 494 nm and 522 nm (absorption and emission, respectively), using a high-resolution bioimaging platform (QIClick-F-CLR-12 Digital CCD camera, Q-imaging, Microvisions Instruments, Evry, France).

Megakaryocyte capture

We define captured megakaryocytes by surface adherent megakaryocytes, independently of their translocation velocity (including non-moving cells) and elongation process. We define elongated megakaryocytes by surface adherent and elongated megakaryocytes, which include megakaryocytes starting their reorganization in order to form their beads-on-a-thread structure as well as megakaryocytes already presenting the specific necklace structure. We evaluated the megakaryocyte capture of megakaryocyte suspensions introduced in the microfluidic device presenting system geometry 1 or 2 described in **Figure 15** and **Figure 16.** Results are shown in **Figure 17 and 18.** Density of captured megakaryocytes is very high at the entrance of the textured area ($\sigma$ ~ 300 to 500 $mm^{-2}$) but this density decreases exponentially along the device at a fixed p ($\sigma < 50$ $mm^{-2}$ at the end of the textured area). In comparison, when cells experience a gradient of posts, the density of adherent megakaryocytes increases at each transition (corresponding to a decrease of the distance p). The density of cells is maintained and can be higher at the end of the textured area (~100 $mm^{-2}$ for "gradient geometry 1").

For a specific megakaryocytes culture and megakaryocyte suspension concentration, we observed that megakaryocyte density was higher at the entrance and lower along the device for a fixed distance p = 85 μm, phenomenon called the axial dependency. This density inequality is reduced when a higher distance p is used at the entrance (p = 120 μm for "gradient geometry 2" in **Figure 17**). The inventors found that increasing the post density along the channel partially compensates the effect of the axial dependency and ensure a more homogeneous megakaryocytes distribution along the device.

We evaluated the megakaryocytes capture according to the different megakaryocytes suspensions introduced in the microfluidic device. Results are shown in **Figure 1.** Density of captured and elongated megakaryocytes has been measured and, in absence of ROCK inhibitor during the culture stage, we observed a sharp density difference between devices receiving megakaryocytes suspension supplemented or not with ROCK inhibitor. The presence of ROCK inhibitor in the suspension during the fluidic process seems to enhance the elongation process since elongated megakaryocytes density is 2.98 ± 0.28 fold more important when ROCK inhibitor is added, as well as the megakaryocytes capture since captured megakaryocytes density is 1.42 ± 0.13 fold more important when ROCK inhibitor is added. Similar effects are observed when ROCK inhibitor is added during the culture stage.

Effect of the protein surface coating

On the vascular endothelial cells, VWF allows translocation of circulating platelets when subjected to high shear rates (>1000 $s^{-1}$) through binding of their GPIb receptors (Huizinga et al, Science, vol.297, n°5584, p.1176-1179, 2002). In vitro, the adsorption of VWF allows megakaryocytes, platelets and proplatelets to translocate on the PDMS and glass surface (Dunois-Lardé et al, Blood, vol.114, n°9, p.1875-1883, 2009). We compared the effect of VWF and BSA coating the channel surface on the adhesion of megakaryocytes with ROCK inhibitor added at day 8 of the culture as well as during the two hours of the microfluidic perfusion.

We performed in parallel one experiment coated with VWF and one experiment coated with BSA. We compared the surface density of megakaryocytes at t = 35 min.

Results are shown in **Figure 2.** When coated with VWF, elongated megakaryocytes density is 7.72 ± 1.32 fold more important that when coated with BSA, while captured megakaryocytes density is 5.86 ± 1 fold more important. VWF sharply increases the density of adherent and elongated cells in channels.

Megakaryocyte ruptures and platelet release

We observed platelet shedding from surface adherent megakaryocytes. When megakaryocytes are translocating on the channel walls, they establish transient interactions with VWF on the wall surface, which progressively lead to morphologic changes until platelet shedding from megakaryocytes. Shedding occurs when both elongation and cell body are translocating. This process is described in the above-cited publication of Dunois-Lardé and in the international patent application WO 2010/06382311. After a rupture, both entities continue translocating on the wall surface.

Shedding also occurs when the cell body translocates until being trapped around or behind a pillar. On the time scale of several minutes, the megakaryocyte undergoes morphological changes leading to the formation of an elongation, which adopted a beads-on-a-thread structure, as previously reported in the above-cited publication of Dunois-Lardé. In addition, instead of full cell contacts with the coated surface, some elongations appeared to be freely moving (dangling) together with the flow, although the cells remained in the same position by at least one point of contact. As the size of the bead-on-a-thread elongation grows, some ruptures occur, releasing platelets and/or proplatelets, as shown in **Figure 3.** No differences in the elongation process as well as the beads-on-a-thread structure formation have been observed when ROCK inhibitor is added in the megakaryocytes suspension. However, we observed a significant higher platelet release when ROCK inhibitor was introduced in the megakaryocytes suspension during the perfusion stage and absent during the culture stage. The hemocytometer counting of platelet production from different conditions was measured. Ratio of platelet production (experimental conditions vs twenty-four static without ROCK inhibitor platelet production) was calculated and is shown in **Figure 4.** Platelets production is significantly higher with presence of shear stress through the microfluidic device and even higher when ROCK inhibitor is added in megakaryocytes suspension for the two hours of the microfluidic perfusion. Produced platelets in the fluidic device that received a megakaryocytes suspension with ROCK inhibitor displayed platelets production that was significantly more important in comparison to the fluidic device that received a megakaryocytes suspension without ROCK inhibitor. A similar tendency is observed when ROCK inhibitor is added during the culture stage.

Other megakaryocyte modulator compounds have been tested for their effect on the quantity and quality of the produced platelets, when specifically added at the perfusion stage in an *ex vivo* method for producing platelet according to the present invention.

Nicotinamide, an inhibitor of the sirtuin pathway, was introduced in the megakaryocyte suspension during the 2 hours of perfusion in the microfluidic device. No differences in the elongation process as well as the beads-on-a-thread structure formation have been observed. However, we observed higher platelet release when nicotinamide was introduced in the megakaryocyte suspension during the perfusion stage and absent during the culture stage (**Figure 10 (A)**). The hemocytometer counting of platelet production from different conditions was measured. Produced platelets in the fluidic device that received a megakaryocyte suspension with nicotinamide displayed platelet production that was more important in comparison to the fluidic device that received a megakaryocyte suspension without nicotinamide. A similar tendency is observed when nicotinamide is also added during the culture stage.

Similar results were observed when blebbistatin (**Figure 11**) or latrunculin (**Figure 12**) were introduced in the megakaryocyte suspension during the perfusion stage and without or with previous introduction during the culture stage.

**Figure 14** shows the effect of the shear stress, at an earlier day than the platelet production day, on the final platelet production. In other words, megakaryocyte suspension was introduced in the VWF-coated microfluidic device at day 10 of culture. After 2 hours of circulation in this device, remaining megakaryocytes, meaning non-captured megakaryocytes, were back in culture for two additional days. Then at day 12, they were re-introduced in a fresh VWF-coated microfluidic device and platelet production was compared with production from megakaryocyte suspension introduced once in the VWF-coated microfluidic device, at day 12. As shown in **Figure 14 (A),** higher platelet release was quantified when megakaryocyte suspension had already circulated into a VWF-coated microfluidic device. This production also showed a higher proportion of $CD41^+/CD42^+$ platelets (**Figure 14** (**B**)).

<u>Characterization of platelet produced in the fluidic device</u>

After two hours of perfusion, produced platelets in the fluidic device that received a megakaryocytes suspension with ROCK inhibitor displayed several characteristics that were better in comparison to those of platelets produced in the fluidic device that received a megakaryocytes suspension without ROCK inhibitor. As shown on **Figure 5,** proportion of platelets expressing CD42b receptor was higher when produced by the fluidic device that received a megakaryocytes suspension with ROCK inhibitor. They also showed similar characteristics that were comparable to those of natural platelets, i.e. platelets isolated from blood (**Figure 6**). Interestingly, **in Figure 7,** upon TRAP-6 stimulation, platelets from megakaryocytes suspension with ROCK inhibitor show a tubulin conformation typical of a transient activation, whereas actin filaments are reorganized as thrombin-activated platelets isolated from blood. They seem to be able to go back in a resting conformation, which is not observed in samples from the fluidic control without addition of the ROCK inhibitor. Without any stimulation, **Figure 7** shows that the tubulin ring characteristic of platelets is present in the samples collected from the fluidic device but not from the static samples. In fact, static samples show presence of filopodia and lamellipodia in TRAP-6 presence and absence. Upon TRAP-6 stimulation, platelets produced in microfluidic device were able to undergo activation features similar to those known to characterize blood platelets, such as to increase their levels of binding of the

PAC1 monoclonal antibody specific of the activated conformation of the αIIbβ3 receptor. According to **Figure 8 and 9,** platelets produced from megakaryocytes suspension with or without ROCK inhibitor both show an activated conformation of the αIIbβ3 receptors upon TRAP-6 stimulation.

Other megakaryocyte modulator compounds have been tested such as nicotinamide (**Figure 10** (**B-C**)), blebbistatin (**Figure 11** (**B-C**)) or latrunculin (**Figure 12** (**B-C**)), and showed a higher proportion of $CD41^+/CD42b^+$ platelet when produced by the fluidic device that received a megakaryocyte suspension with nicotinamide, blebbistatin or latrunculin during the 2 hours of perfusion, without or with previous introduction during the culture stage. Similar tendency was observed for two additional experiments for each tested compound (data not shown).

A metalloproteinase inhibitor, GM6001, has been tested for its effect on improving the final yield of $CD42b^+$ platelet. CD42b receptor has been shown to be unstable on the surface of platelets produced ex vivo. It may be due to the cleavage by metalloproteinase. Addition of the broad-range metalloproteinase inhibitor GM6001 during the culture as well as during the 2 hours perfusion stage increased the proportion of platelet $CD42b^+$. As shown in **Figure 13,** proportion of platelets expressing CD42b receptor was higher when produced in the fluidic device that received a megakaryocyte suspension supplemented with GM6001 for 2 hours. A similar effect was detected when the megakaryocyte suspension was cultured, at the later stage, with GM6001. Similar tendency was observed for two additional experiments (data not shown).

## Claims

1. An ex vivo method for producing platelets from megakaryocytes, said method comprising:

    (a) providing a fluidic device comprising a production chamber including at least one channel delimited by (i) non-porous walls, (ii) one inlet opening at one end and (iii) one outlet opening at the other end;
    (b) introducing a suspension of cells comprising megakaryocytes into the inlet opening of the channel;
    (c) subjecting said suspension to a flow from the inlet to the outlet of said channel, under a shear rate suitable for elongation and fragmentation of the megakaryocyte and platelet release in said channel,
    (d) collecting platelets at the outlet of the channel,

    wherein at least one megakaryocyte modulator compound is added to said suspension of cells, said at least one megakaryocyte modulator compound being selected from:

    i. an inhibitor of the Rho/ROCK pathway capable of increasing megakaryocytes ploidy,
    ii. nicotinamide,
    iii. blebbistatin,
    iv. latrunculin, and
    v. GM6001.

2. The method of Claim 1, wherein said channel of the fluidic device is coated with a ligand with binding affinity for megakaryocytes, e.g., (i) von Willebrand factor (VWF) or its functional variants, (ii) polypeptides comprising fragments of von Willebrand factor, (iii) fibrinogen or (iv) fibronectin.

3. The method of any one of claims 1-2, wherein said suspension of cells of megakaryocytes is obtained by the following steps:

    (i) providing megakaryocyte progenitor and/or stem cells, for example CD34+ cells from human umbilical cord blood;
    (ii) culturing said megakaryocyte progenitor and/or stem cells for expanding the cells and differentiating the expanded cells into megakaryocytes;
    (iii) washing the cells to remove the culture medium;
    (iv) re- suspending the cells in a perfusion medium;

    wherein said megakaryocyte modulator compound is added after the washing step (iii).

4. The method according to any one of Claims 1-3, wherein the cells are collected at the outlet of the channel and reintroduced into the inlet of the channel for reperfusion in the channel and platelet production.

5. The method according to Claim 4, wherein prior to reintroduction into the inlet of the channel, said collected cells are

cultured for further maturation into megakaryocyte.

6. The method of any one of Claims 1-5, wherein said channel of the fluidic device has a substantially square or rectangular section.

7. The method of Claim 6, wherein said channel has a height H that is comprised between 5 μm and 1 mm, preferably a height comprised between 25 μm and 100 μm and a width comprised between 100 μm and 5 mm, preferably a width comprised between 300 μm and 800 μm.

8. The method of claim any one of Claims 1-7, wherein said production chamber comprises a plurality of parallel channels, for example between 2 channels and $10^6$ channels.

9. The method according to any one of Claims 1-8, wherein said suspension of cells is subjected to a flow in the channel under a shear rate of at least 300 $s^{-1}$, preferably at least 600 $s^{-1}$, and more preferably at least 1000 $s^{-1}$, for example between 300 $s^{-1}$ and 5000 $s^{-1}$, or between 600 $s^{-1}$ and 2400 $s^{-1}$.

10. The method according to Claim 3, wherein the at least one megakaryocyte modulator compound is comprised in the perfusion medium.

11. The method of claim 1, wherein said an inhibitor of the Rho/ROCK pathway is hydroxyfasudil.

## Patentansprüche

1. Ex-vivo-Verfahren zur Herstellung von Thrombozyten aus Megakaryozyten, wobei das Verfahren umfasst:

    (a) Bereitstellen einer Fluidvorrichtung, die eine Produktionskammer aufweist, die mindestens einen Kanal umfasst, der begrenzt ist durch (i) nicht poröse Wände, (ii) eine Einlassöffnung an einem Ende und (iii) eine Auslassöffnung am anderen Ende;
    (b) Einbringen einer Suspension von Zellen, die Megakaryozyten enthält, in die Einlassöffnung des Kanals;
    (c) Unterwerfen der Suspension einem Fluss vom Einlass zum Auslass des Kanals bei einer Schergeschwindigkeit, die für die Elongation und Fragmentierung der Megakaryozyten und die Freisetzung von Thrombozyten in dem Kanal geeignet ist,
    (d) Sammeln der Thrombozyten am Auslass des Kanals,

    wobei mindestens eine Megakaryozyten-Modulatorverbindung zu der Suspension von Zellen hinzugefügt wird, wobei die mindestens eine Megakaryozyten-Modulatorverbindung ausgewählt ist aus:

    i. einem Inhibitor des Rho/ROCK-Signalwegs, der den Ploidiegrad der Megakaryozyten erhöhen kann,
    ii. Nicotinamid,
    iii. Blebbistatin,
    iv. Latrunkulin und
    v. GM6001.

2. Verfahren nach Anspruch 1, wobei der Kanal der Fluidvorrichtung mit einem Liganden beschichtet ist, der eine Bindungsaffinität für Megakaryozyten aufweist, z. B. (i) ein Von-Willebrand-Faktor (VWF) oder dessen funktionelle Varianten, (ii) Polypeptide, die Fragmente des Von-Willebrand-Faktors enthalten, (iii) Fibrinogen oder (iv) Fibronektin.

3. Verfahren nach einem der Ansprüche 1 bis 2, wobei die Suspension von Megakaryozytenzellen durch die folgenden Schritte erhalten wird:

    (i) Bereitstellen von Megakaryozytenvorläufer- und/oder Stammzellen, beispielsweise CD34+-Zellen aus menschlichem Nabelschnurblut;
    (ii) Kultivieren der Megakaryozytenvorläufer- und/oder Stammzellen, um die Zellen zu vermehren und die vermehrten Zellen zu Megakaryozyten zu differenzieren;
    (iii) Waschen der Zellen, um das Kulturmedium zu entfernen;
    (iv) Resuspendieren der Zellen in einem Perfusionsmedium;

wobei die Megakaryozyten-Modulatorverbindung nach dem Waschschritt (iii) zugegeben wird.

4. Verfahren nach einem der Ansprüche 1 bis 3, wobei die Zellen am Auslass des Kanals gesammelt und zum Zwecke der Reperfusion im Kanal und der Thrombozytenproduktion wieder in den Einlass des Kanals einbracht werden.

5. Verfahren nach Anspruch 4, wobei die gesammelten Zellen vor dem Wiedereinbringen in den Einlass des Kanals zur weiteren Reifung zu Megakaryozyten kultiviert werden.

6. Verfahren nach einem der Ansprüche 1 bis 5, wobei der Kanal der Fluidvorrichtung einen im Wesentlichen quadratischen oder rechteckigen Querschnitt aufweist.

7. Verfahren nach Anspruch 6, wobei der Kanal eine Höhe H zwischen 5 μm und 1 mm, vorzugsweise eine Höhe zwischen 25 μm und 100 μm, und eine Breite zwischen 100 μm und 5 mm, vorzugsweise eine Breite zwischen 300 μm und 800 μm, aufweist.

8. Verfahren nach einem der Ansprüche 1 bis 7, wobei die Produktionskammer eine Vielzahl paralleler Kanäle umfasst, beispielsweise zwischen 2 Kanäle und $10^6$ Kanäle.

9. Verfahren nach einem der Ansprüche 1 bis 8, wobei die Suspension von Zellen in dem Kanal einer Strömung unterworfen wird, und zwar mit einer Schergeschwindigkeit von mindestens 300 $s^{-1}$, vorzugsweise mindestens 600 $s^{-1}$ und noch bevorzugter mindestens 1000 $s^{-1}$, beispielsweise zwischen 300 $s^{-1}$ und 5000 $s^{-1}$ oder zwischen 600 $s^{-1}$ und 2400 $s^{-1}$.

10. Verfahren nach Anspruch 3, wobei die mindestens eine Megakaryozyten-Modulatorverbindung in dem Perfusionsmedium enthalten ist.

11. Verfahren nach Anspruch 1, wobei es sich bei dem Inhibitor des Rho/ROCK-Signalwegs um Hydroxyfasudil handelt.

## Revendications

1. Procédé ex vivo pour la production de thrombocytes à partir de mégacaryocytes, ledit procédé comprenant :

(a) la fourniture d'un dispositif fluidique comprenant une chambre de production ayant au moins un canal délimité par (i) des parois non poreuses, (ii) une ouverture d'entrée à une extrémité et (iii) une ouverture de sortie à l'autre extrémité ;
(b) l'introduction d'une suspension de cellules contenant des mégacaryocytes dans l'ouverture d'entrée du canal ;
(c) la soumission de la suspension à un écoulement de l'entrée vers la sortie du canal à une vitesse de cisaillement appropriée pour l'élongation et la fragmentation des mégacaryocytes et la libération de thrombocytes dans le canal,
(d) la collecte des thrombocytes à la sortie du canal,

au moins un composé modulateur des mégacaryocytes étant ajouté à la suspension de cellules, ledit au moins un composé modulateur des mégacaryocytes étant choisi parmi :

i. un inhibiteur de la voie de signalisation Rho/ROCK capable d'augmenter le degré de ploïdie des mégacaryocytes,
ii. la nicotinamide,
iii. la blebbistatine,
iv. la latrunculine et
v. le GM6001.

2. Le procédé selon la revendication 1, sachant que le canal du dispositif fluidique est recouvert d'un ligand qui présente une affinité de liaison pour les mégacaryocytes, lequel peut être par exemple (i) un facteur Von Willebrand (VWF) ou ses variants fonctionnels, (ii) des polypeptides contenant des fragments du facteur Von Willebrand, (iii) du fibrinogène ou (iv) de la fibronectine.

3. Le procédé selon l'une des revendications 1 à 2, sachant que la suspension de cellules mégacaryocytaires est obtenue par les étapes suivantes :

(i) la fourniture de précurseurs mégacaryocytaires et/ou de cellules souches, par exemple des cellules CD34+ provenant de sang de cordon ombilical humain ;
(ii) la culture des précurseurs mégacaryocytaires et/ou des cellules souches afin de multiplier les cellules et de différencier les cellules multipliées en mégacaryocytes ;
(iii) le lavage des cellules pour éliminer le milieu de culture ;
(iv) la re-suspension des cellules dans un milieu de perfusion ;

le composé modulateur des mégacaryocytes étant ajouté après l'étape de lavage (iii).

4. Le procédé selon l'une des revendications 1 à 3, sachant que les cellules sont collectées à la sortie du canal et réintroduites dans l'entrée du canal à des fins de reperfusion dans le canal et de production de thrombocytes.

5. Le procédé selon la revendication 4, sachant que les cellules collectées sont cultivées pour mûrir en mégacaryocytes avant d'être réintroduites dans l'entrée du canal.

6. Le procédé selon l'une des revendications 1 à 5, sachant que le canal du dispositif fluidique présente une section transversale sensiblement carrée ou rectangulaire.

7. Le procédé selon la revendication 6, sachant que le canal présente une hauteur H comprise entre 5 $\mu$m et 1 mm, de préférence comprise entre 25 $\mu$m et 100 $\mu$m, et une largeur comprise entre 100 $\mu$m et 5 mm, de préférence comprise entre 300 $\mu$m et 800 $\mu$m.

8. Le procédé selon l'une des revendications 1 à 7, sachant que la chambre de production comprend une pluralité de canaux parallèles, par exemple entre 2 et $10^6$ canaux.

9. Le procédé selon l'une des revendications 1 à 8, sachant que la suspension de cellules dans le canal est soumise à un écoulement avec une vitesse de cisaillement d'au moins 300 $s^{-1}$, de préférence d'au moins 600 $s^{-1}$ et encore plus préférablement d'au moins 1000 $s^{-1}$, par exemple comprise entre 300 $s^{-1}$ et 5000 $s^{-1}$ ou entre 600 $s^{-1}$ et 2400 $s^{-1}$.

10. Le procédé selon la revendication 3, sachant que ledit au moins un composé modulateur des mégacaryocytes est contenu dans le milieu de perfusion.

11. Le procédé selon la revendication 1, sachant que l'inhibiteur de la voie de signalisation Rho/ROCK est l'hydroxy-fasudil.

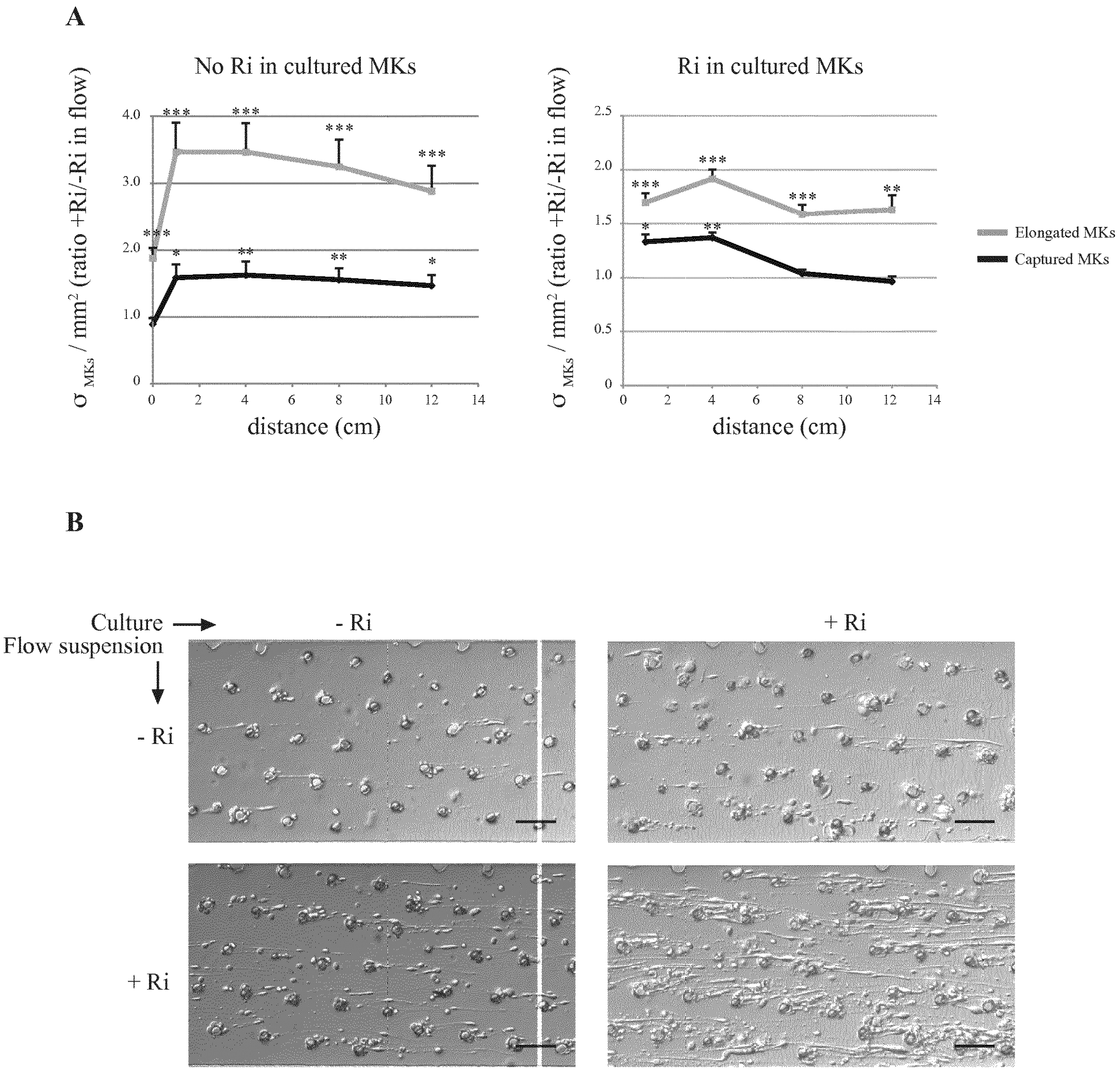
A
No Ri in cultured MKs
Ri in cultured MKs
σ MKs / mm² (ratio +Ri/-Ri in flow)
distance (cm)
Elongated MKs
Captured MKs
B
Culture
Flow suspension
- Ri
+ Ri
- Ri
+ Ri

FIGURE 1

A

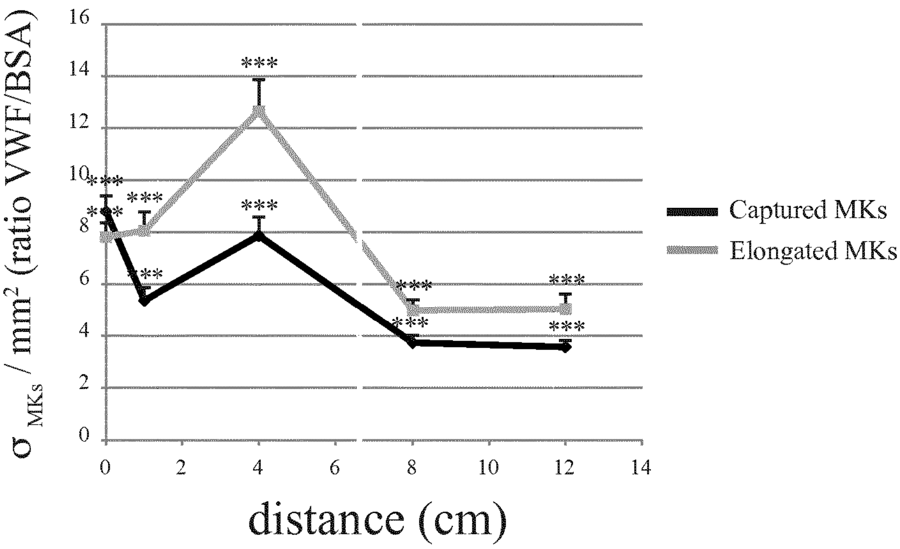
σ MKs / mm² (ratio VWF/BSA)
distance (cm)
Captured MKs
Elongated MKs
***

B

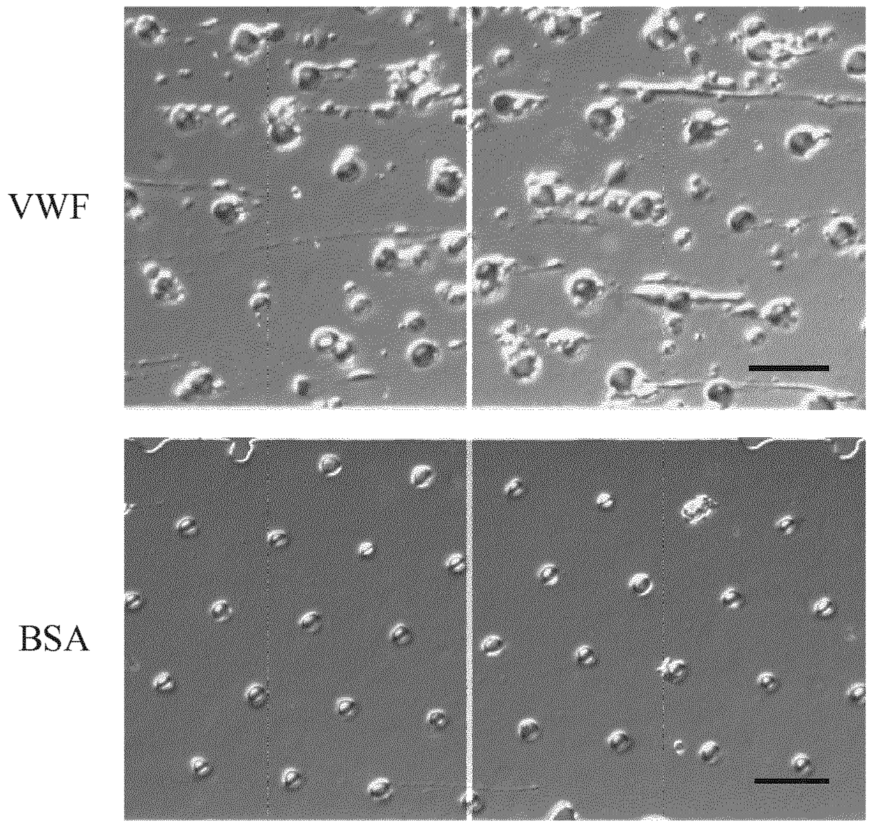
VWF
BSA

**FIGURE 2**

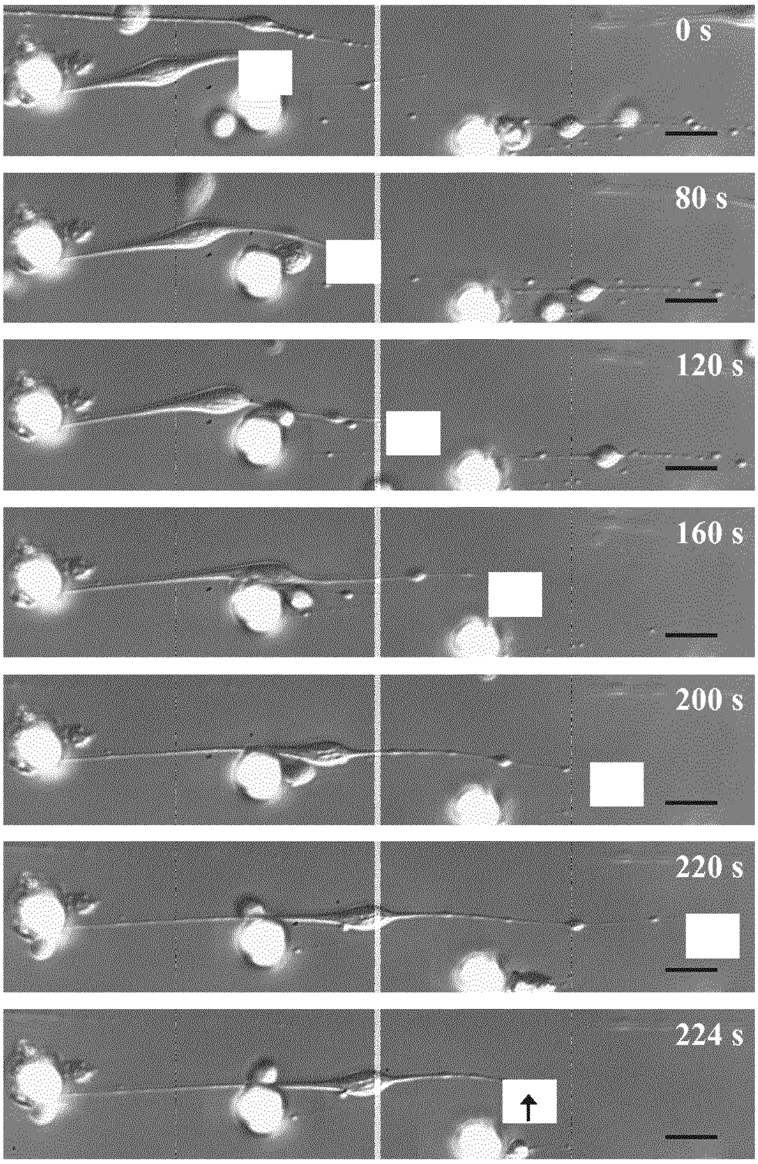


FIGURE 3

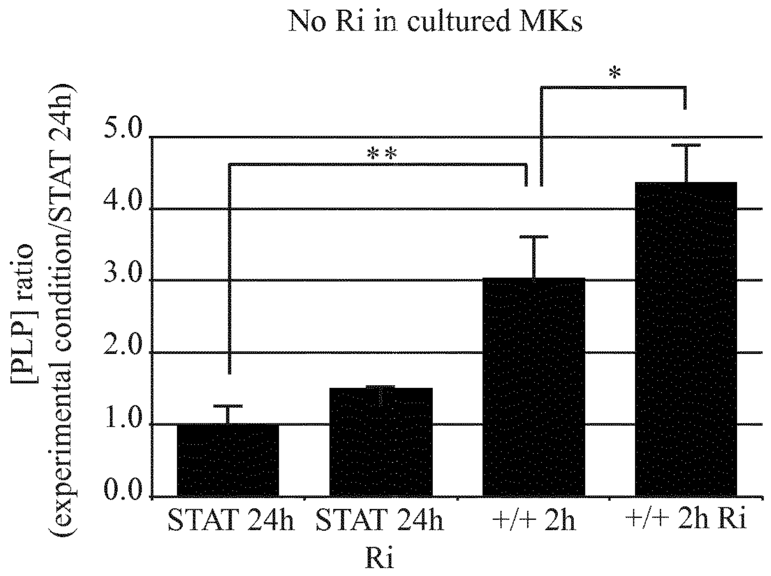
No Ri in cultured MKs
[PLP] ratio
(experimental condition/STAT 24h)
5.0
4.0
3.0
2.0
1.0
0.0
**
*
STAT 24h
STAT 24h
Ri
+/+ 2h
+/+ 2h Ri

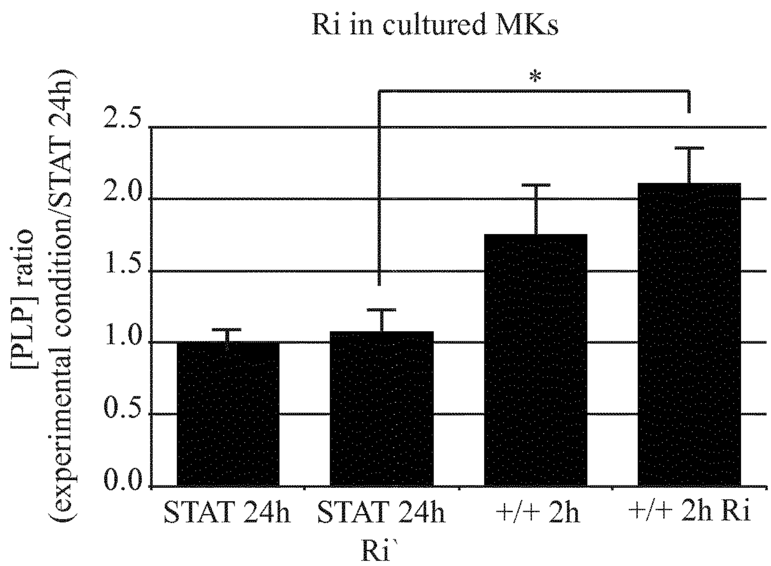
Ri in cultured MKs
[PLP] ratio
(experimental condition/STAT 24h)
2.5
2.0
1.5
1.0
0.5
0.0
*
STAT 24h
STAT 24h
Ri`
+/+ 2h
+/+ 2h Ri

FIGURE 4

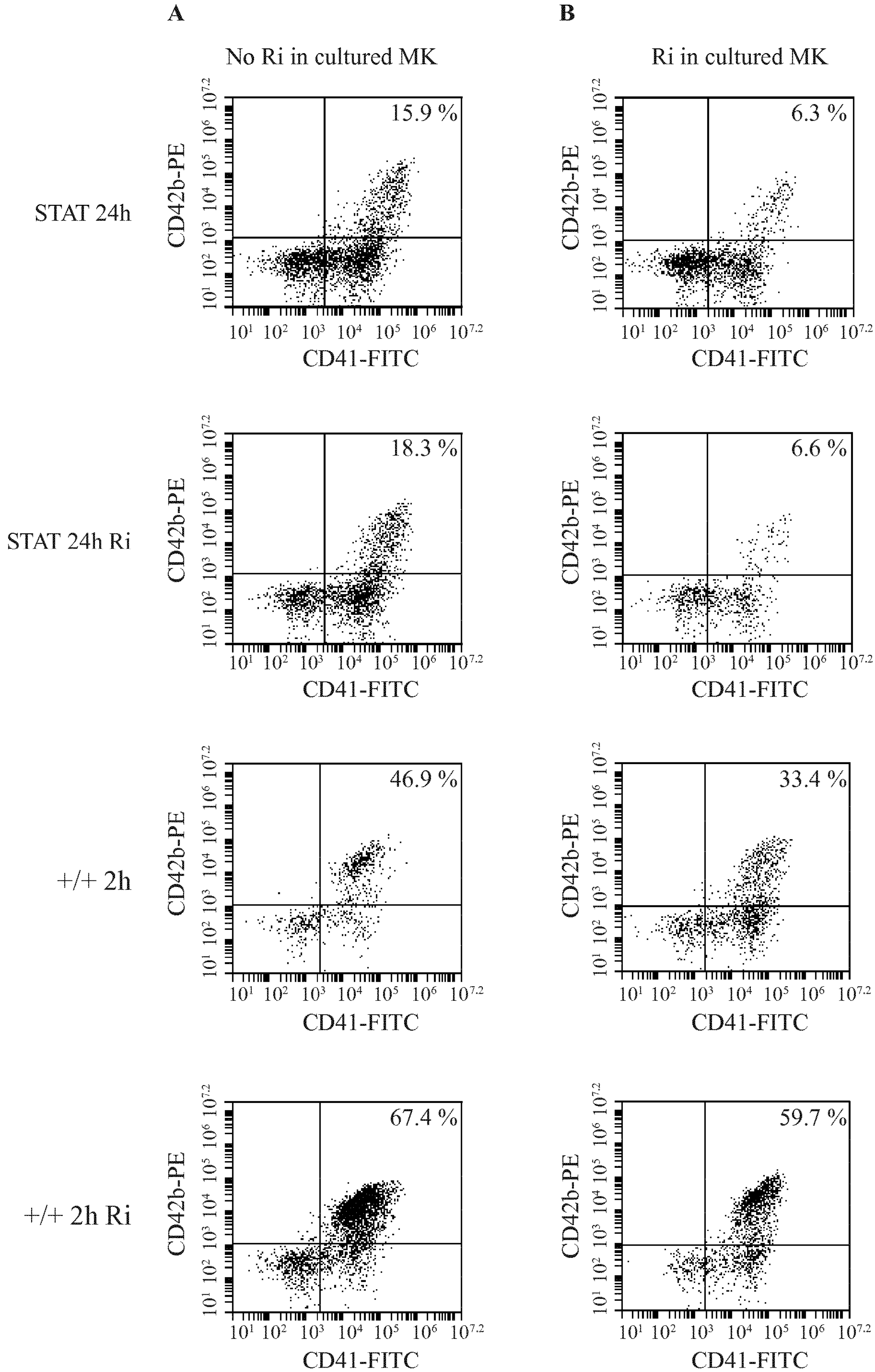
A
B
No Ri in cultured MK
Ri in cultured MK
STAT 24h
STAT 24h Ri
+/+ 2h
+/+ 2h Ri
15.9 %
6.3 %
18.3 %
6.6 %
46.9 %
33.4 %
67.4 %
59.7 %
CD42b-PE
CD41-FITC

FIGURE 5

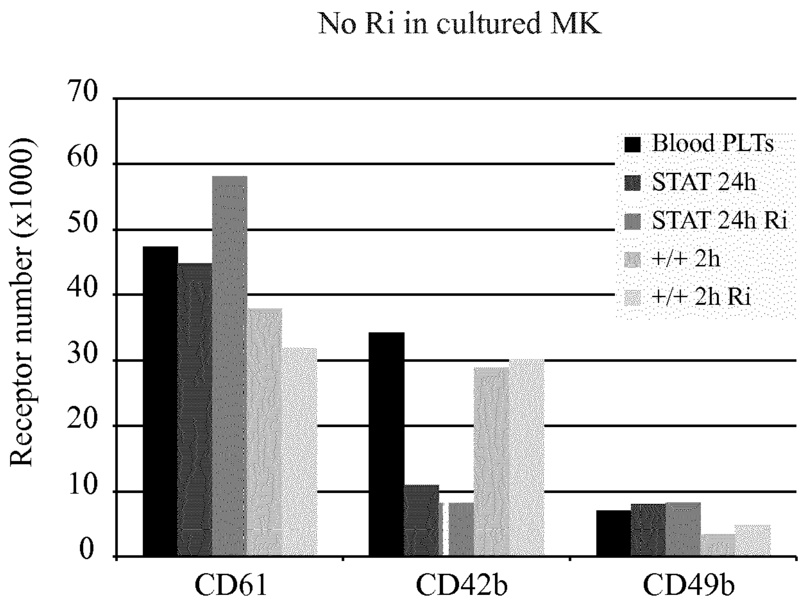
No Ri in cultured MK
Receptor number (x1000)
70
60
50
40
30
20
10
0
Blood PLTs
STAT 24h
STAT 24h Ri
+/+ 2h
+/+ 2h Ri
CD61
CD42b
CD49b

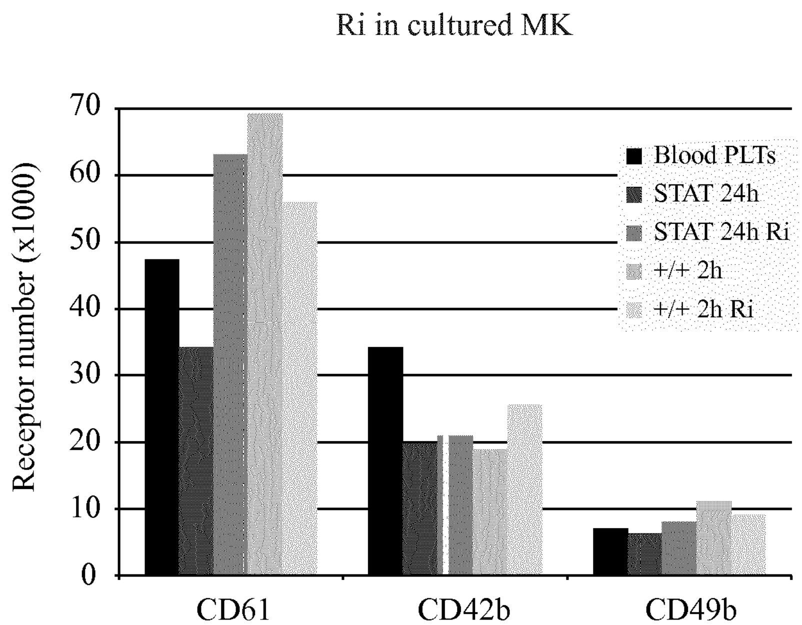
Ri in cultured MK
Receptor number (x1000)
70
60
50
40
30
20
10
0
Blood PLTs
STAT 24h
STAT 24h Ri
+/+ 2h
+/+ 2h Ri
CD61
CD42b
CD49b

**FIGURE 6**

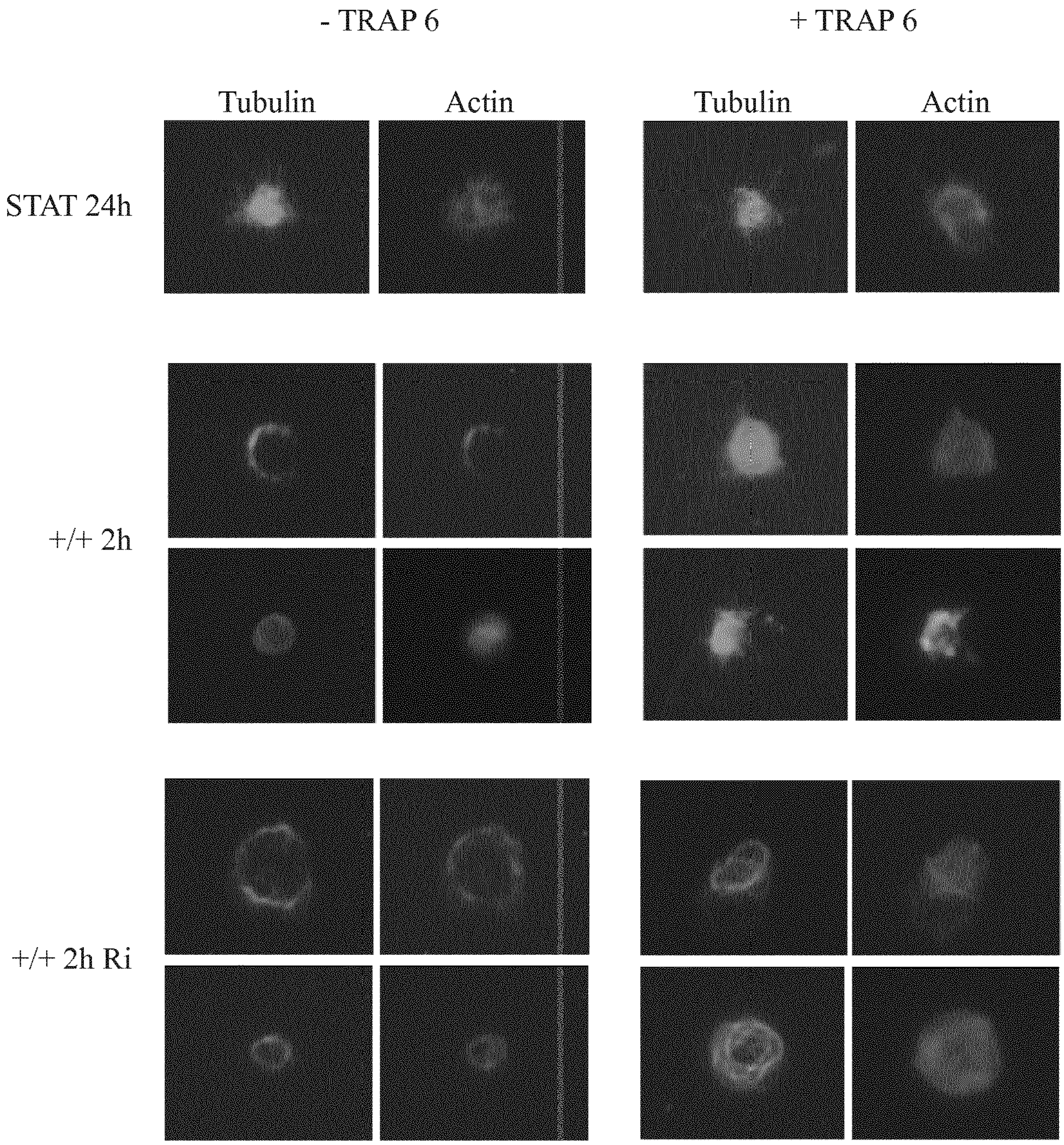


FIGURE 7

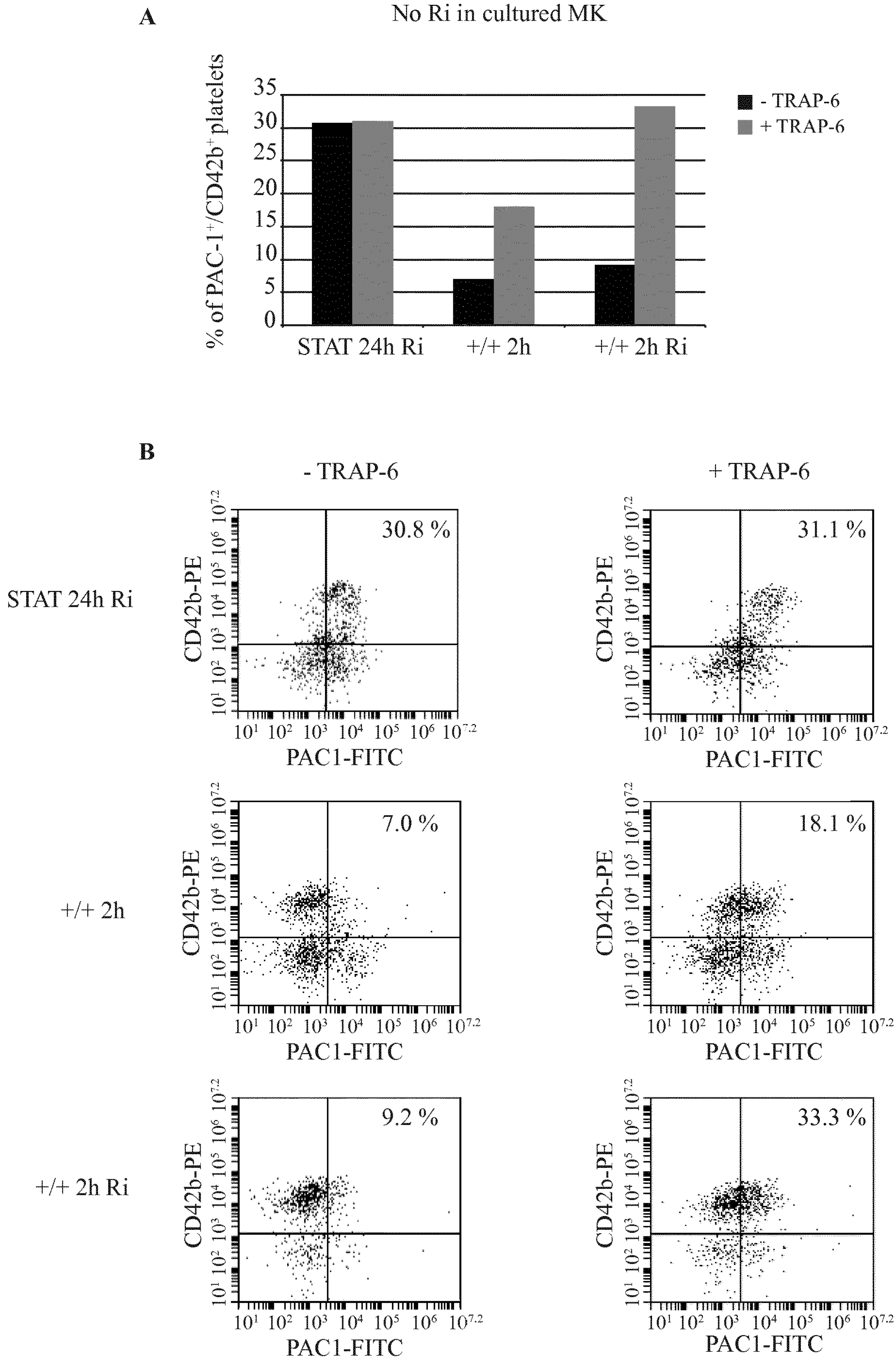
A
No Ri in cultured MK
% of PAC-1+/CD42b+ platelets
35
30
25
20
15
10
5
0
STAT 24h Ri
+/+ 2h
+/+ 2h Ri
- TRAP-6
+ TRAP-6
B
- TRAP-6
+ TRAP-6
STAT 24h Ri
+/+ 2h
+/+ 2h Ri
30.8 %
31.1 %
7.0 %
18.1 %
9.2 %
33.3 %
CD42b-PE
PAC1-FITC

**FIGURE 8**

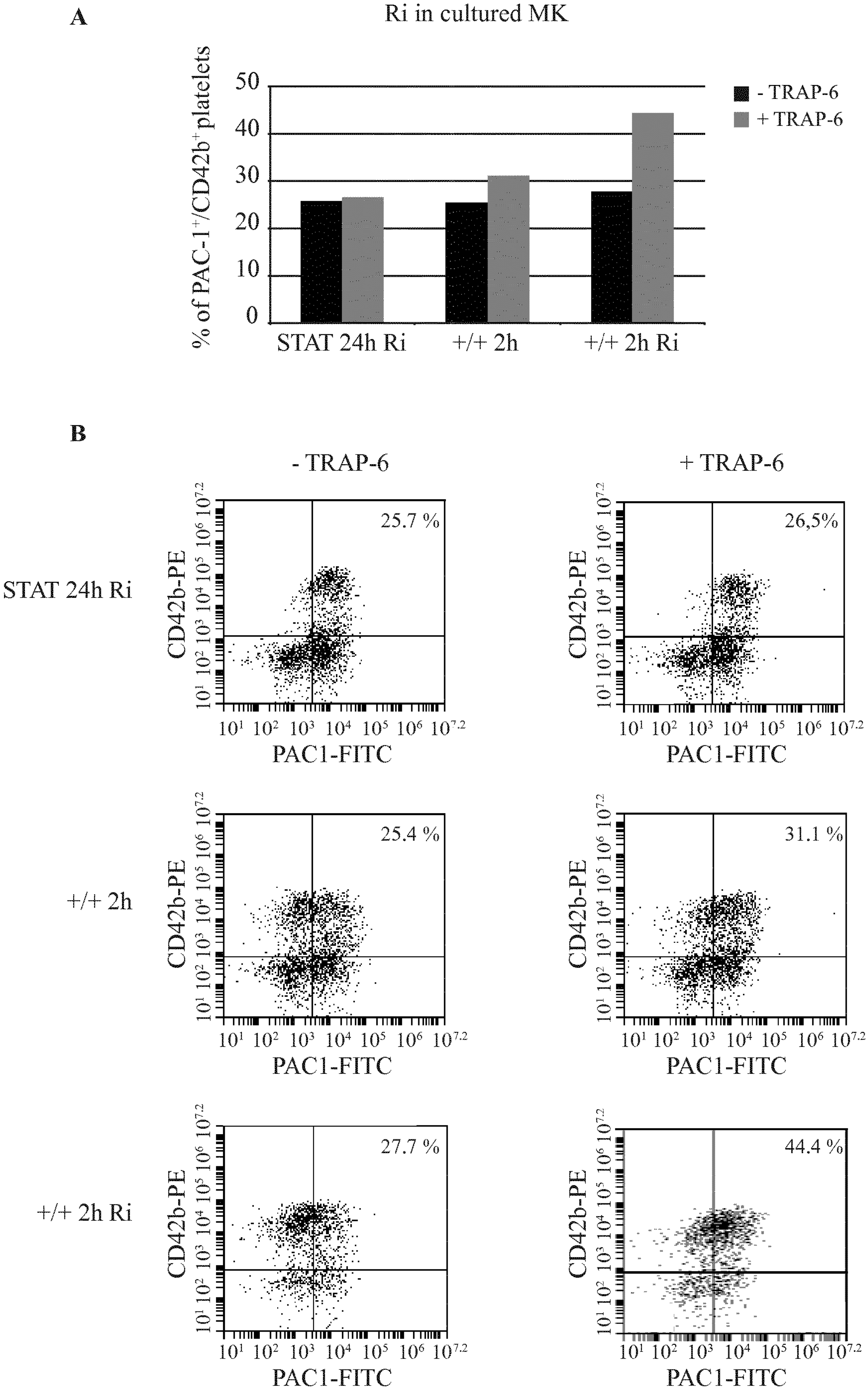
A
Ri in cultured MK
% of PAC-1+/CD42b+ platelets
50
40
30
20
10
0
- TRAP-6
+ TRAP-6
STAT 24h Ri
+/+ 2h
+/+ 2h Ri
B
- TRAP-6
+ TRAP-6
STAT 24h Ri
+/+ 2h
+/+ 2h Ri
25.7 %
26,5%
25.4 %
31.1 %
27.7 %
44.4 %
CD42b-PE
PAC1-FITC

**FIGURE 9**

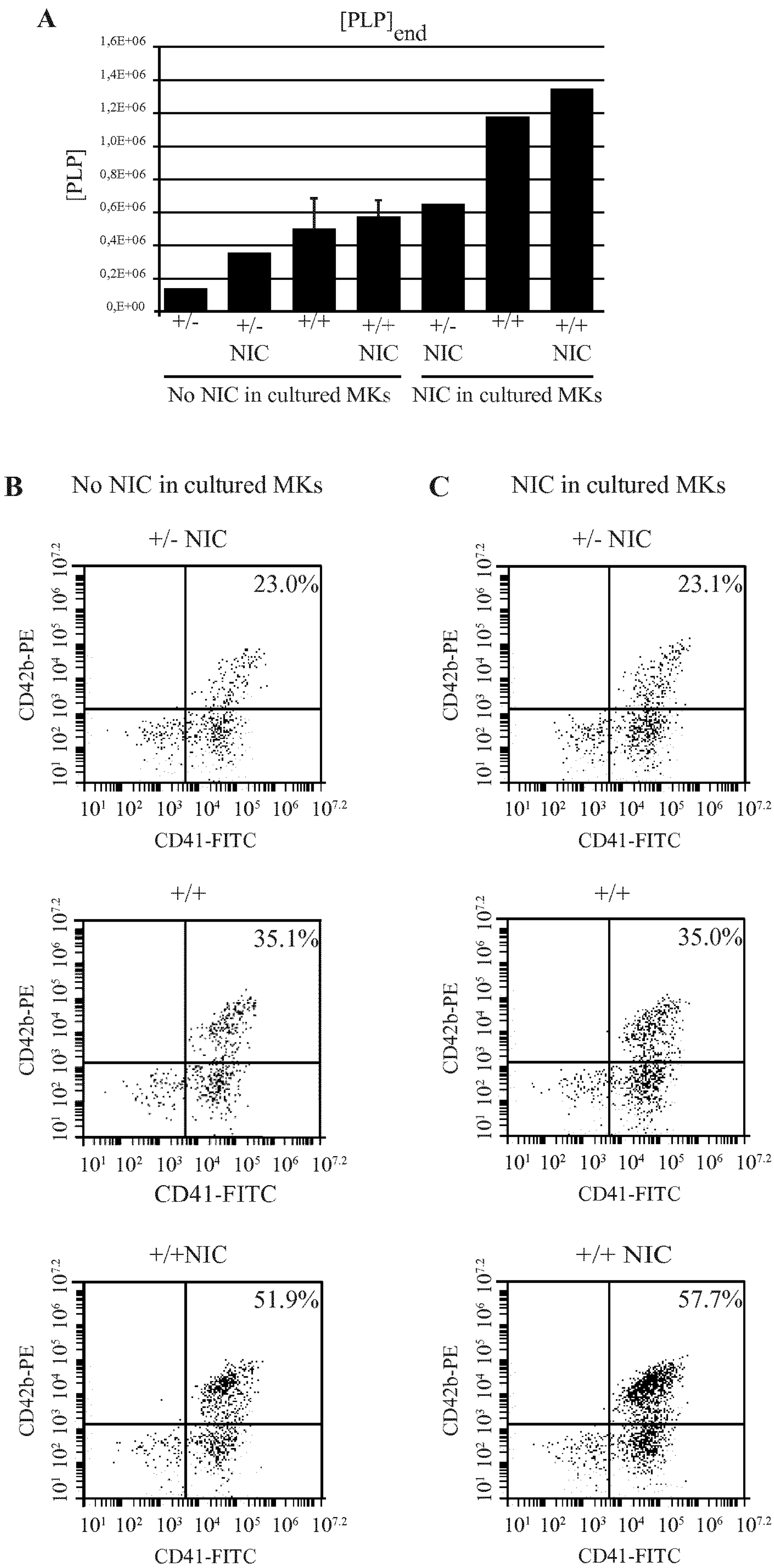
A
[PLP]end
[PLP]
1,6E+06
1,4E+06
1,2E+06
1,0E+06
0,8E+06
0,6E+06
0,4E+06
0,2E+06
0,E+00
+/-
+/- NIC
+/+
+/+ NIC
+/- NIC
+/+
+/+ NIC
No NIC in cultured MKs
NIC in cultured MKs
B
No NIC in cultured MKs
C
NIC in cultured MKs
+/- NIC
23.0%
23.1%
+/+
35.1%
35.0%
+/+NIC
51.9%
+/+ NIC
57.7%
CD42b-PE
CD41-FITC

FIGURE 10

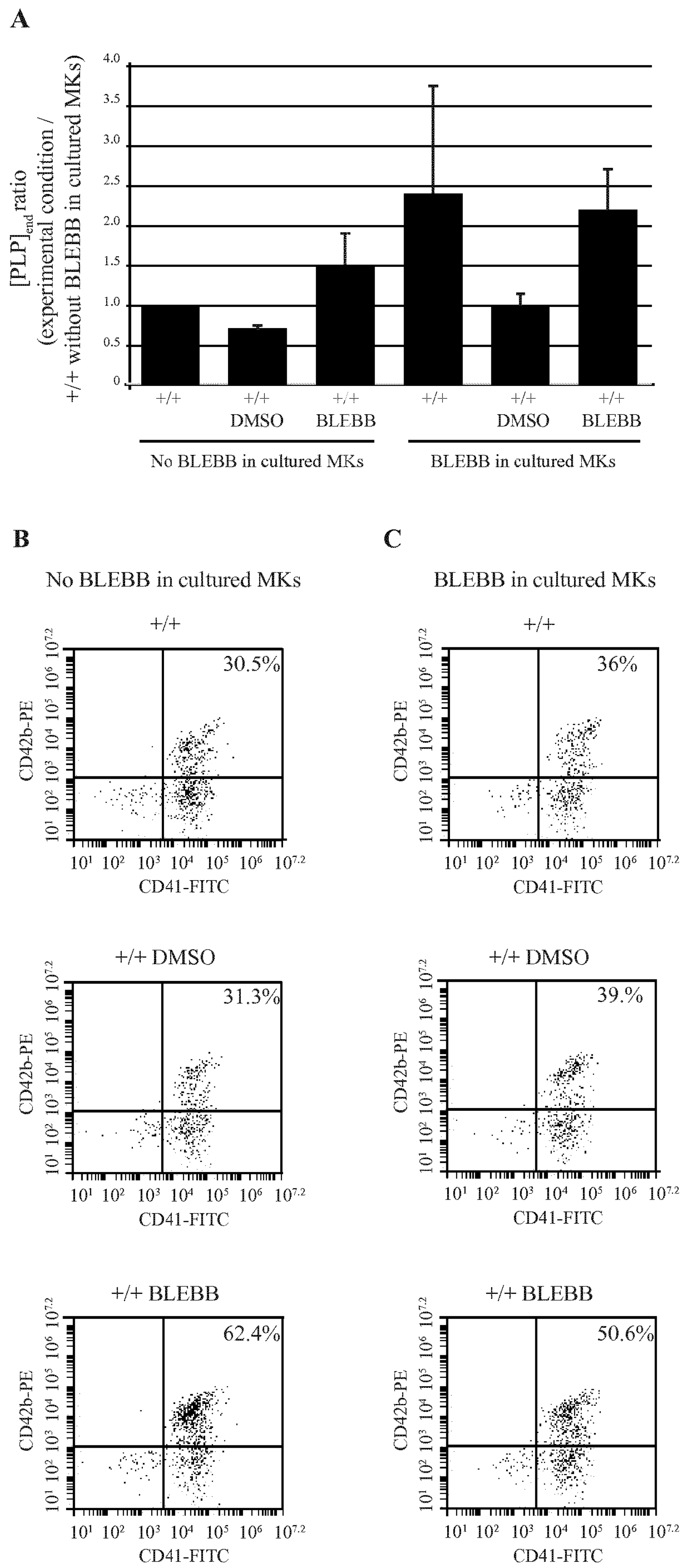
A
[PLP]end ratio (experimental condition / +/+ without BLEBB in cultured MKs)
4.0
3.5
3.0
2.5
2.0
1.5
1.0
0.5
0
+/+
+/+ DMSO
+/+ BLEBB
+/+
+/+ DMSO
+/+ BLEBB
No BLEBB in cultured MKs
BLEBB in cultured MKs
B
No BLEBB in cultured MKs
C
BLEBB in cultured MKs
+/+
30.5%
36%
+/+ DMSO
31.3%
39.%
+/+ BLEBB
62.4%
50.6%
CD42b-PE
CD41-FITC
10¹ 10² 10³ 10⁴ 10⁵ 10⁶ 10^7.2

FIGURE 11

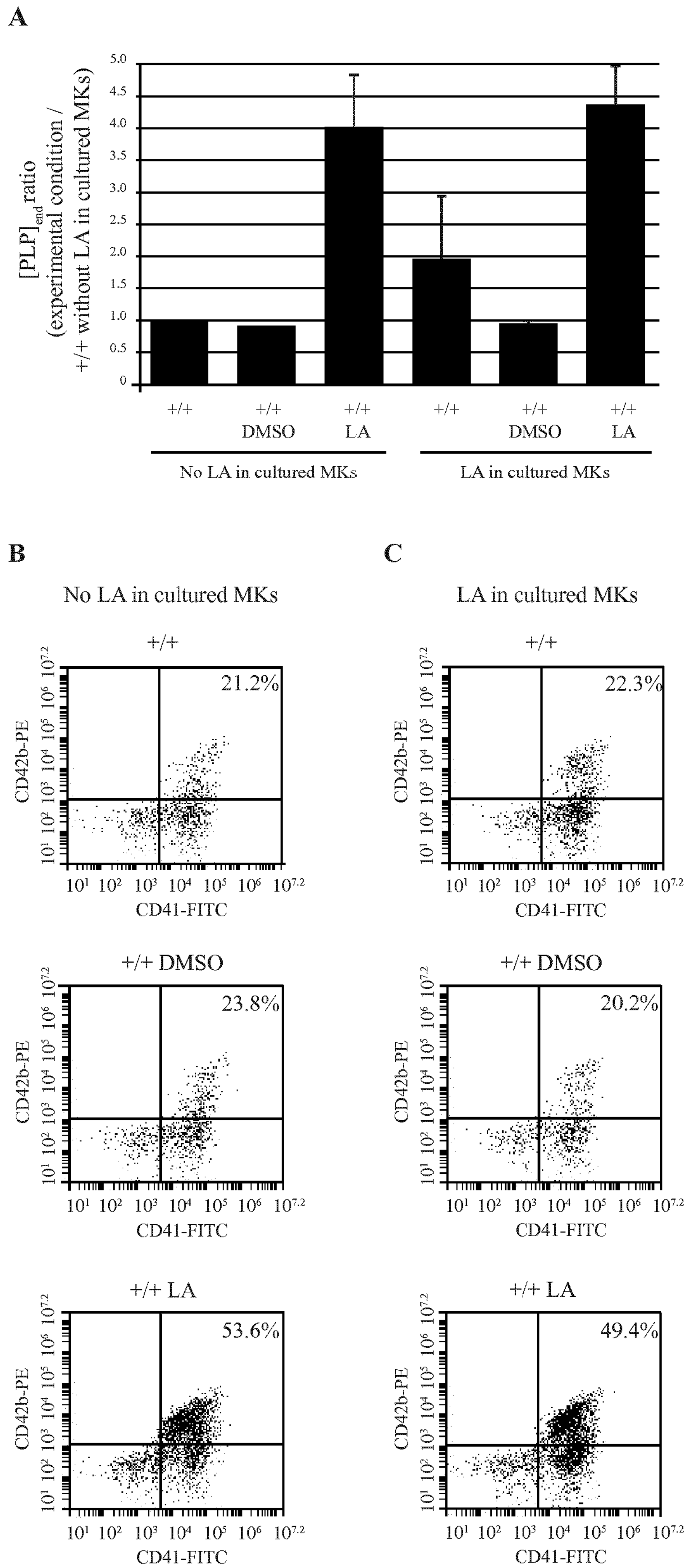
A
[PLP]end ratio (experimental condition / +/+ without LA in cultured MKs)
5.0
4.5
4.0
3.5
3.0
2.5
2.0
1.5
1.0
0.5
0
+/+
+/+ DMSO
+/+ LA
+/+
+/+ DMSO
+/+ LA
No LA in cultured MKs
LA in cultured MKs
B
C
No LA in cultured MKs
LA in cultured MKs
+/+
21.2%
22.3%
+/+ DMSO
23.8%
20.2%
+/+ LA
53.6%
49.4%
CD42b-PE
CD41-FITC
10¹ 10² 10³ 10⁴ 10⁵ 10⁶ 10⁷·²

FIGURE 12

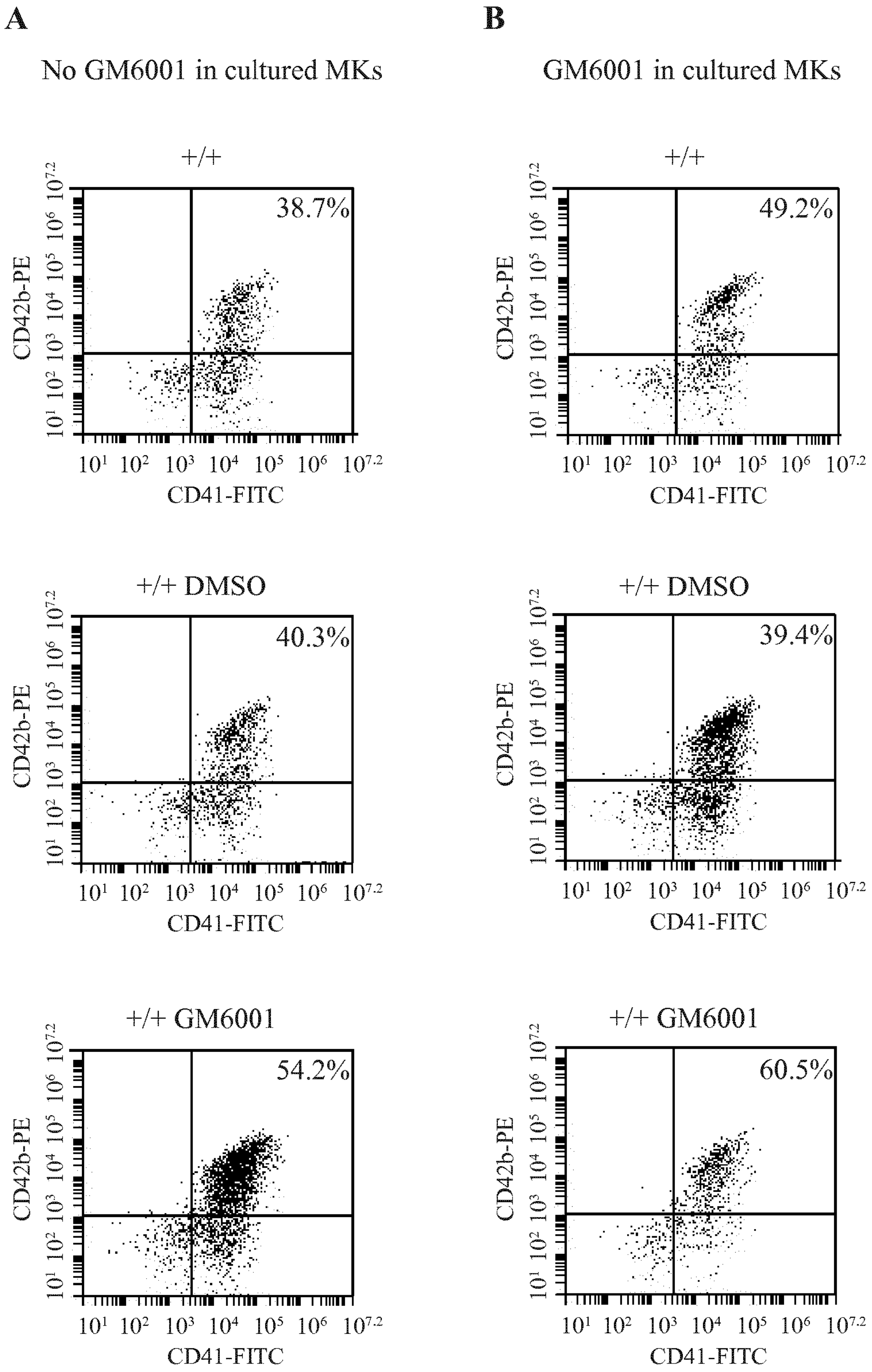
A
No GM6001 in cultured MKs
B
GM6001 in cultured MKs
+/+
38.7%
+/+
49.2%
+/+ DMSO
40.3%
+/+ DMSO
39.4%
+/+ GM6001
54.2%
+/+ GM6001
60.5%
CD42b-PE
CD41-FITC
10¹ 10² 10³ 10⁴ 10⁵ 10⁶ 10^7.2

**FIGURE 13**

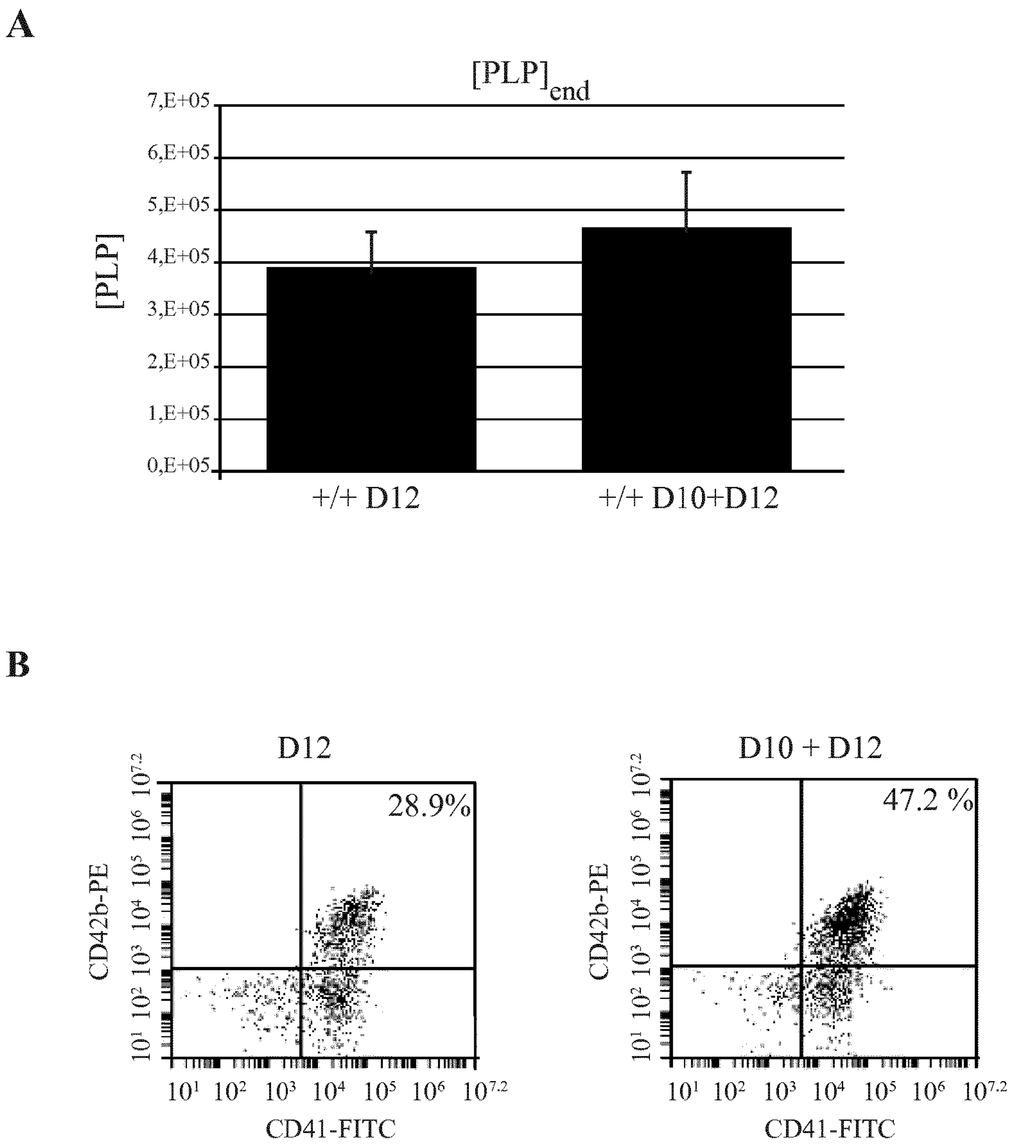
A
[PLP]end
7,E+05
6,E+05
5,E+05
4,E+05
3,E+05
2,E+05
1,E+05
0,E+05
[PLP]
+/+ D12
+/+ D10+D12
B
D12
28.9%
D10 + D12
47.2 %
CD42b-PE
CD41-FITC

**FIGURE 14**

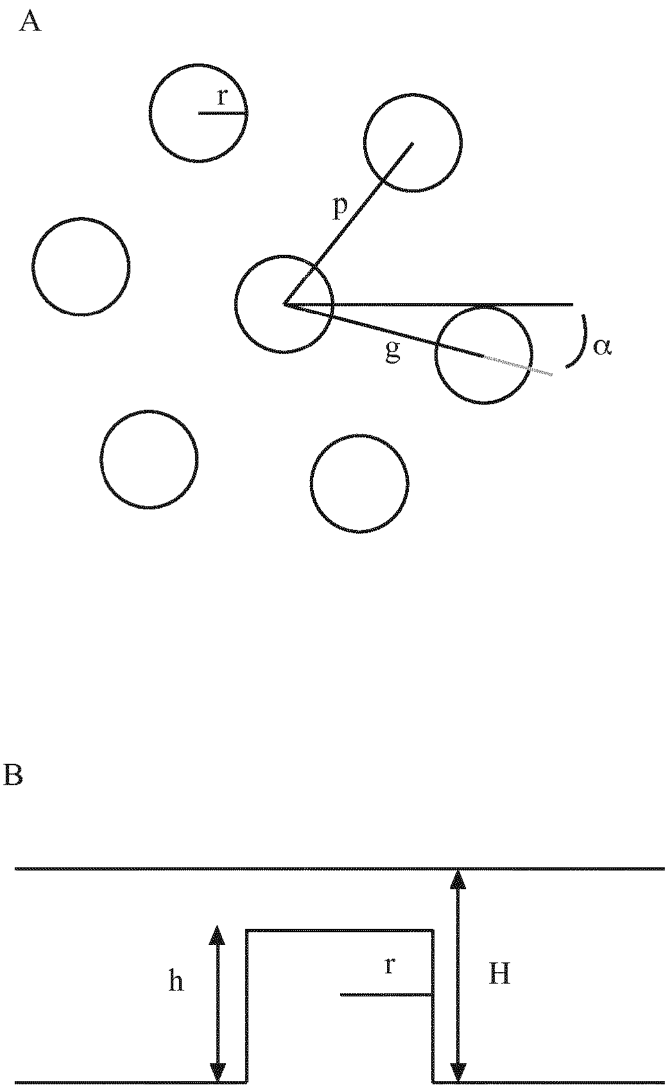


FIGURE 15

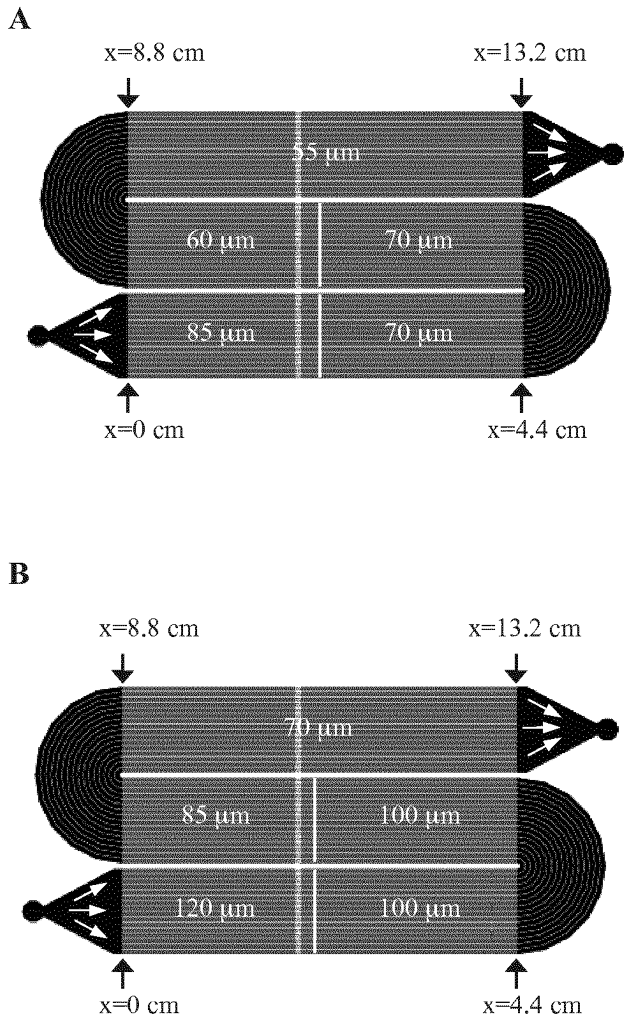
A
x=8.8 cm
x=13.2 cm
55 µm
60 µm
70 µm
85 µm
70 µm
x=0 cm
x=4.4 cm
B
x=8.8 cm
x=13.2 cm
70 µm
85 µm
100 µm
120 µm
100 µm
x=0 cm
x=4.4 cm

FIGURE 16

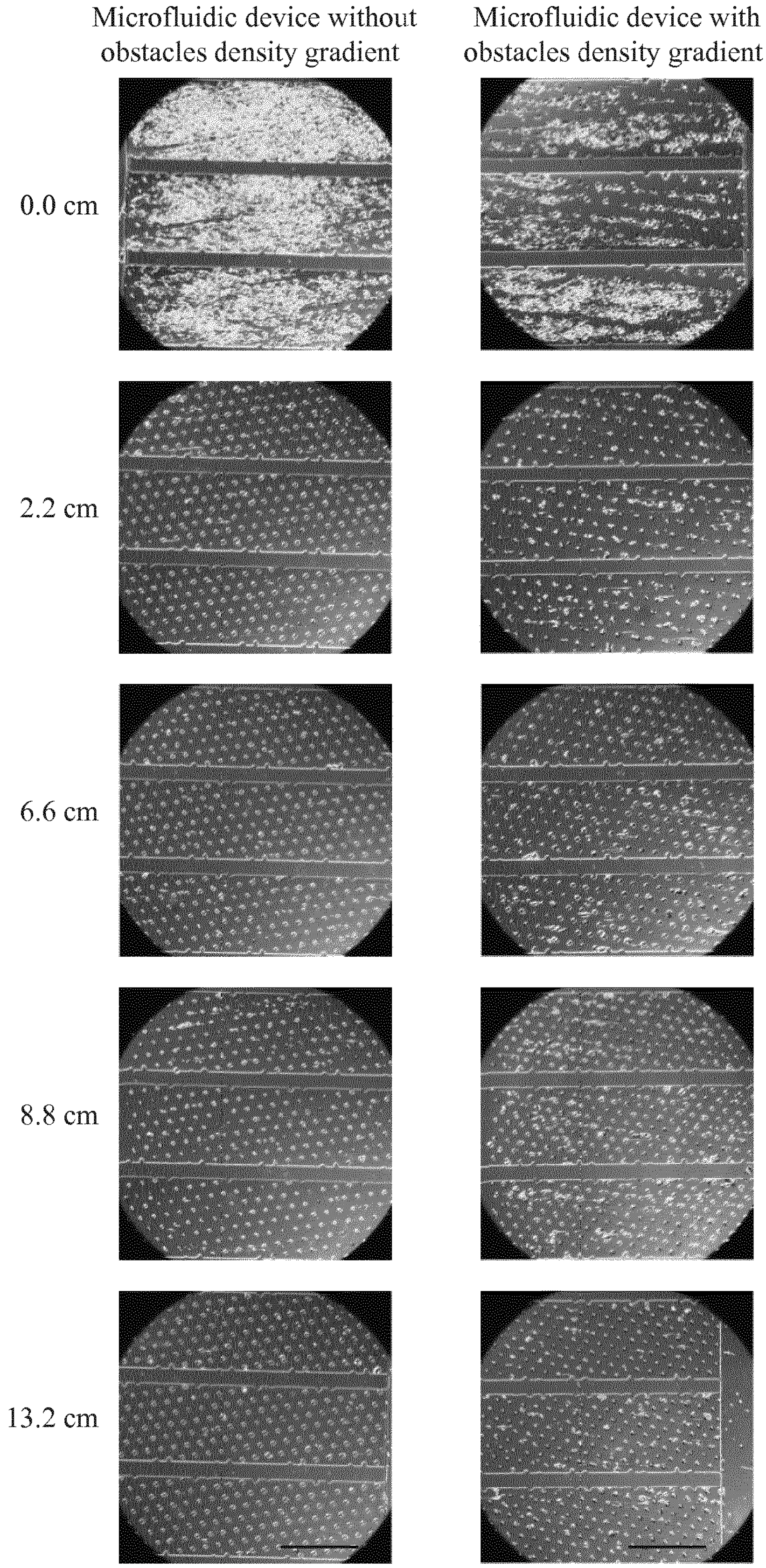
Microfluidic device without obstacles density gradient
Microfluidic device with obstacles density gradient
0.0 cm
2.2 cm
6.6 cm
8.8 cm
13.2 cm

FIGURE 17

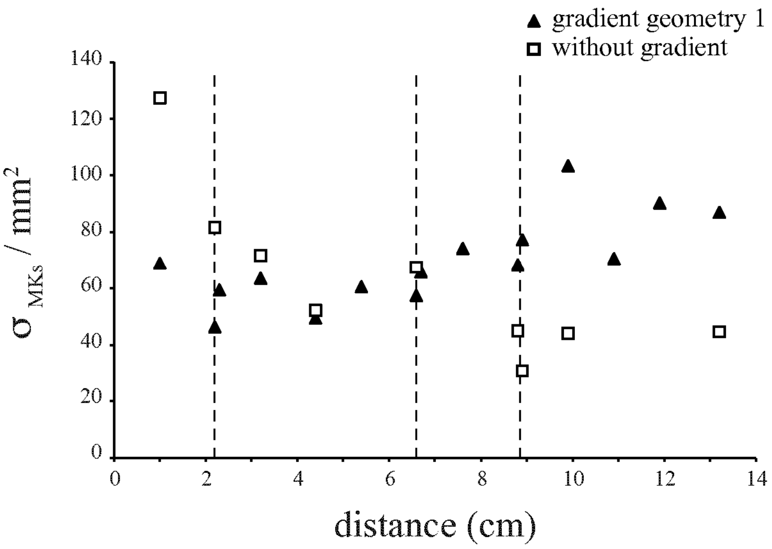
gradient geometry 1
without gradient
σ MKs / mm²
distance (cm)
0
20
40
60
80
100
120
140
0
2
4
6
8
10
12
14

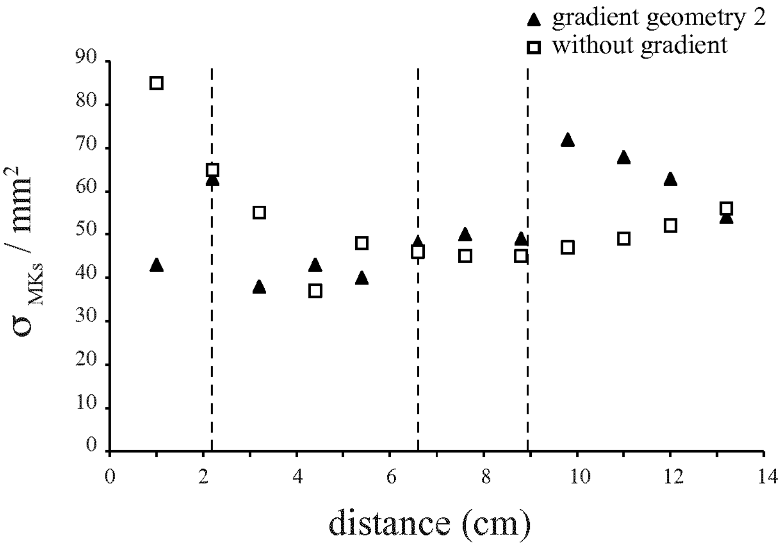
gradient geometry 2
without gradient
σ MKs / mm²
distance (cm)
0
10
20
30
40
50
60
70
80
90
0
2
4
6
8
10
12
14

**FIGURE 18**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2014074905 A
- US 8093266 B
- US 8541449 B
- US 8604218 B
- US 8697911 B
- US 8796458 B
- US 9000154 B
- US 2656508 A
- WO 201006382311 A


### Non-patent literature cited in the description


- **PALLOTTA et al.** *Tissue Engineering: Part C*, 2011, vol. 17 (12), 1223-1232
- **DI BUDUO et al.** *Blood*, 2015, vol. 125 (14), 2254-2264
- **NAKAGAWA et al.** *Experimental Hematology*, 2013, vol. 41 (8), 742-748
- **THON et al.** *Blood*, 2014, vol. 124 (12), 1857-1867
- **CHANG et al.** *Blood*, 2007, vol. 109 (10), 4229-4236
- **AVANZI et al.** *British Journal of Haematology*, 2014, vol. 164, 867-876
- **BALDUINI et al.** *J Thromb Haemost*, 2008, vol. 6, 1900-1907
- **TAKAYAMA et al.** *The Journal of Experimental Medicine*, 2010, vol. 207, 2817-2830
- **NAKAMURA et al.** *Cell Stem Cell*, 2014, vol. 14, 1-14
- **FENG et al.** *Stem Cell Reports*, 2014, vol. 3, 1-15
- **RAVID et al.** *J Cell physiology*, 2002, vol. 190, 7-20
- *CHEMICAL ABSTRACTS*, 103745-39-7
- *CHEMICAL ABSTRACTS*, 155558-32-0
- **XU et al.** *PNAS*, 2010, vol. 107 (18), 8129-8134
- **LORDIER et al.** *Blood*, 2008, vol. 112 (8), 3164-3174
- **SHI et al.** *The Journal of Clinical Investigation*, 2014, vol. 124 (9), 3757-3766
- **EGOT et al.** *Thrombosis and Haemostasis*, 2013, vol. 110, 1215-1222
- **LUKAS et al.** *Journal of Medicinal Chemistry*, 1999, vol. 42 (5), 910-919
- **GIAMMONA et al.** *British Journal of Haematology*, 2006, vol. 135, 554-566
- **GIAMMONA et al.** *Exp Hematol.*, 2009, vol. 37 (11), 1340-1352
- *CHEMICAL ABSTRACTS*, 14513-15-6
- **VILLALBA et al.** *Biofactors*, 2012, vol. 38 (5), 349-359
- **SHIN et al.** *PNAS*, 2011, vol. 108 (28), 11458-11463
- **LIMOUZE et al.** *J Muscle Res Cell Motil*, 2004, vol. 25 (4-5), 337-341
- *CHEMICAL ABSTRACTS*, 674289-55-5
- **BOND et al.** *Future Med Chem*, 2013, vol. 5 (1), 41-52
- *CHEMICAL ABSTRACTS*, 76343-93-6
- **AVANZI et al.** *PLoS ONE*, vol. 10 (4), e0125057
- *CHEMICAL ABSTRACTS*, 76343-94-7
- *CHEMICAL ABSTRACTS*, 105304-96-9
- **E. MYERS** ; **W. MILLER**. *Comput. Appl. Biosci.*, 1988, vol. 4, 1 1-17
- **NEEDLEMAN** ; **WUNSCH**. *J. Mol. Biol.*, 1970, vol. 48, 443-453
- **M. LARKIN et al.** *Bioinformatics*, 2007, vol. 23, 2947-2948
- **D. HIGGINS** ; **P. SHARP**. *Gene*, 1988, vol. 73, 237-244
- **MARTIN et al.** *Journal of Thrombosis and Haemostasis*, 2007, vol. 5, 1363-1370
- **XIA et al.** *Annual Review of Materials Science*, 1998, vol. 28, 153-184
- **STROOCK et al.** *Science*, 2002, vol. 295 (5555), 647-651
- **TAKAGI et al.** *Lab Chip*, 2005, vol. 5 (7), 778
- **L. R. HUANG et al.** *Science*, 2004, vol. 304 (987)
- **POIRAULT-CHASSAC et al.** Notch/Delta4 signaling inhibits human megakaryocytic terminal differentiation. *Blood*, 2010, vol. 116 (25), 5670-5678
- **DUNOIS-LARDÉ et al.** Exposure of human megakaryocytes to high shear rates accelerates platelet production. *Blood*, 2009, vol. 114 (9), 1875-1883

- **ROBERT A** ; **CORTIN V** ; **GARNIER A** ; **PINEAULT N.** Megakaryocyte and platelet production from human cord blood stem cells.. *Methods Mol Biol.*, 2012, vol. 788, 219-47
- **XIA et al.** Soft lithography. *Annual Review of Materials Science*, 1998, vol. 28 (1), 153-184
- **L. R. HUANG et al.** *Science*, 2004, vol. 304, 987
- **ANTFOLK et al.** *Lab Chip*, 2014, vol. 14, 2791-2799
- **HUIZINGA et al.** *Science*, 2002, vol. 297 (5584), 1176-1179
- **DUNOIS-LARDÉ et al.** *Blood*, 2009, vol. 114 (9), 1875-1883